(19) Europäisches Patentamt
European Patent Office
Office européen des brevets

(11) **EP 4 012 016 A1**

(12) **EUROPEAN PATENT APPLICATION**
published in accordance with Art. 153(4) EPC

(43) Date of publication:
**15.06.2022 Bulletin 2022/24**

(21) Application number: **20853067.5**

(22) Date of filing: **11.08.2020**

(51) International Patent Classification (IPC):
*C12M 3/00* (2006.01)       *C12N 5/00* (2006.01)
*C12N 5/0775* (2010.01)       *C12N 5/20* (2006.01)

(52) Cooperative Patent Classification (CPC):
**C12M 3/00; C12N 5/00**

(86) International application number:
**PCT/JP2020/030614**

(87) International publication number:
**WO 2021/029409 (18.02.2021 Gazette 2021/07)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **09.08.2019 JP 2019148236**

(71) Applicant: Ube Industries, Ltd.
**Yamaguchi 755-8633 (JP)**

(72) Inventors:
• **HAGIHARA, Masahiko**
  **Ube-shi, Yamaguchi 755-8633 (JP)**
• **HIDAKA, Motonobu**
  **Ube-shi, Yamaguchi 755-8633 (JP)**
• **HARADA, Takashi**
  **Ube-shi, Yamaguchi 755-8633 (JP)**
• **KONOSHIMA, Hayato**
  **Ube-shi, Yamaguchi 755-8633 (JP)**

(74) Representative: Plougmann Vingtoft a/s
**Strandvejen 70**
**2900 Hellerup (DK)**

(54) **CELL CULTURING METHOD USING SMALL-PIECE POROUS MEMBRANE**

(57) The purpose of the present invention is to provide a cell culturing method using a small-piece polymer porous membrane. The present invention provides a cell culturing method which does not require stirring and comprises applying cells to a small-piece polymer porous membrane having an area of a surface layer A or B of at most 4 mm2, and culturing the cells. The small-piece polymer porous membrane has a characteristic three-dimensional structure, and a medium can be more easily supplied and recovered by utilizing the property that small-piece polymer porous membranes are dispersed in a cell culture solution and/or small-piece polymer porous membranes are accumulated in multiple layers, and thus the small-piece polymer porous membranes are dispersed and/or laminated on a bottom part of a cell culture vessel. Consequently, long-term culture and mass culture are achieved, and thus the stable long-term production of exosomes is possible.

FIG. 2

Long-term production of exosomes with small-piece porous membranes

Number of particles / Days

EP 4 012 016 A1

**Description**

FIELD

The present invention relates to cell culturing and a substance production system, and more specifically it relates to a cell culturing method using small-piece polymer porous membranes. The invention further relates to a cell culturing apparatus and kit provided with the small-piece polymer porous membranes.

BACKGROUND

Cell culturing

Cells generally exist as three-dimensional aggregates in the body. However, when cells are cultured in an artificial environment, it is common to use the classical plate culture method in which the cells are cultured two-dimensionally in a manner plated as a monolayer on the bottom of the culturing vessel, or a suspension culture method in which cells are cultured while dispersed in a liquid culture solution. Cells most suited for the plate culture method are cells having relatively high adhesion, but even when such suitable cells are used, differences in the culturing environment can often result in significant changes in the properties of the cells. With suspension culture methods as well, certain cells are suitable while others are not.

With increasing demand for *in vivo* proteins to be used for medical purposes, such as vaccines, enzymes, hormones, antibodies, cytokines and the like, it is becoming increasingly important to achieve mass production of such *in vivo* proteins by cell culturing. In addition, with the trend toward practical implementation of cell grafting for regenerative medicine, greater focus is being directed toward methodologies for efficient and convenient culturing of large volumes of cells. Moreover, in recent years cell culture systems have been developed for efficient production of exosomes designed to be used as diagnostic markers, drug delivery carriers and biological drugs, and which are able to function as bioactive substances.

For suspended cells of *E. coli* and the like, research is being conducted on techniques for mass culturing in large-scale culturing tanks. Mass culturing of suspended cells using large-scale culturing tanks requires large volumes of culture solution and a stirring apparatus.

Increasing focus is also being directed toward research in which substances are produced using adherent cells, as research on such cells continues to progress. When it is attempted to perform mass culturing of adherent cells, the cells will only expand two-dimensionally when the classical plate culture method is employed, and therefore a large culturing area is necessary.

A method using a bioreactor or cell culture support has been reported as a method of culturing large volumes of cells in a three-dimensional environment (NPL 1 and PTL 1). Methods using a bioreactor include a method in which a fibrous material such as a glass fiber material is accumulated in a column, and the cells are continuously cultured in the space to produce a substance (PTL 2). Microcarriers, which are microparticles on which cells can adhere and grow, are being widely studied as typical cell culturing supports (PTLs 3 and 4).

PTL 4 describes an example of such production and teaches that, in cell culturing methods using microcarriers, the most important factor for raising production volume and increasing efficiency is to reach a high-density cell culture. Also important is whether the cells can efficiently and conveniently proliferate, and can be transplanted and seeded onto a microcarrier support. In this regard, in cell culturing systems using microcarriers it is necessary to carry out sufficient agitation and diffusion to avoid causing the microcarriers to aggregate together. Since this requires a volume space allowing adequate agitation and diffusion of the culture solution in which the microcarriers are dispersed, there is a limit to the density at which the cells can be cultured. In order to provide a sufficient supply of nutrients by stirring it is important for the cells to not easily detach, and therefore resistance to shear force is an essential property. Further issues also still remain in terms of volume and efficiency because it is necessary to separate the fine particles with a separable filter in order to separate the microcarriers and the culture solution. Numerous three-dimensional culture techniques have been developed as well, but most of these techniques, such as the hydrogel or spheroid method, are geared toward temporary culturing or evaluation, and no culturing method has yet been reported that allows production of substances for prolonged periods. Stable, long-term culturing techniques are therefore required to produce biopharmaceuticals and bioactive rare substances, or substances such as exosomes. Long-term culturing of primary cells including mesenchymal stem cells has been reported (NPL 2; NPL 3), but this has not been robust as a production method for cell culture systems.

The physiological activity of exosomes is supported by the structures of nucleic acids and proteins controlled by the cell from which they are secreted, and the quality of the exosomes produced varies at each stage of cell culturing including the induction phase, logarithmic growth phase, resting phase and death phase. From the viewpoint of producing exosomes on an industrial scale it is undesirable in terms of quality control for the properties of the exosomes to vary throughout the culturing period, and it is desirable to allow production of exosomes of uniform quality over long periods in already established production systems. The problem of time-dependent variation in exosome quality, in cell culturing using conventional exosome production techniques using such cultured cells, has not yet been properly dealt with, and this has constituted a technical barrier against supply of exosomes of stable quality and establishment of exosome production systems on an industrial scale.

From the viewpoint of oxygen supply in cell culturing, regardless of whether or not a cell culture support is used in the cell culturing and regardless of whether suspended cells or adherent cells are used, supply of oxygen is an essential issue for achieving healthy growth of the cells, with the exception of anaerobic bacteria. For example, when adherent cells are plate cultured using a dish or plate or a chamber, there are restrictions on the proper range for the medium volume with respect to the area of the culturing vessel. Therefore, when an excessive amount of medium has been used, it is often the case that oxygen may be insufficiently supplied to the cells and the low oxygen condition may lead to inhibition or even cell death. Moreover, with spheroid-forming cell groups, an excessively large size is known to result in oxygen deficiency for the cells in the interior (NPL 4). As a solution for the issue of oxygen supply it has been attempted to increase the oxygen concentration by utilizing microbubbles (PTL 5) or to employ methods for uniformly supplying oxygen in microcarrier culturing (PTL 6). For culturing of mesenchymal stem cells, on the other hand, since the cells prefer to grow and proliferate in a low-oxygen environment, complex procedures are necessary to moderate the supply of oxygen during the culturing, depending on the cell type. This has raised demand for development and design of cell culturing methods that allow oxygen supply levels to be easily adjusted by convenient, automatable processes, and that allow culturing of even larger amounts of cells.

The present inventors have previously provided a cell culturing method and cell culturing apparatus using a polymer porous membrane, but the polymer porous membrane is housed in a casing as a cell culture module, which is applied in a cell culturing vessel, cell culturing apparatus, or cell culturing system to prevent deformation of the continuous membranous form of the polymer porous membrane (PTL 7: "Cell culture module"). It is thereby possible to prevent application of stress to the cells grown in the polymer porous membrane, and to inhibit apoptosis, thus allowing stable and large-volume culturing of the cells. However, a cell culturing method using such a cell culture module requires a casing to house the polymer porous membrane, and also has restrictions on the size and strength of the cell culturing vessel in which the casing is situated.

<Polyimide porous membranes>

Polyimide porous membranes have been utilized in the prior art for filters and low permittivity films, and especially for battery-related purposes, such as fuel cell electrolyte membranes and the like. PTLs 8 to 10 describe polyimide porous membranes with numerous macro-voids, having excellent permeability for gases and the like, high porosity, excellent smoothness on both surfaces, relatively high strength and, despite high porosity, also excellent resistance against compression stress in the film thickness direction. All of these are polyimide porous membranes formed using amic acid.

[CITATION LIST]

[PATENT LITERATURE]

[PTL 1] Japanese Unexamined Patent Publication SHO No. 62-065681
[PTL 2] International Patent Publication No. 2008/084857
[PTL 3] Japanese Unexamined Patent Publication HEI No. 7-313151
[PTL 4] International Patent Publication No. 2003/054174
[PTL 5] Japanese Patent Publication No. 5549209
[PTL 6] Japanese Patent Publication No. 5460241
[PTL 7] International Patent Publication No. 2018/021368
[PTL 8] International Patent Publication No. 2010/038873
[PTL 9] Japanese Unexamined Patent Publication No. 2011-219585
[PTL 10] Japanese Unexamined Patent Publication No. 2011-219586

[NON PATENT LITERATURE]

[NPL 1] Ogata et al., Journal of Fermentation and Bioengineering Vol. 77, No. 1, p.46-51 1994

# EP 4 012 016 A1

[NPL 2] Dosh, R. H., et al., Science Reports, volume 9, Article number: 1812 (2019)

[NPL 3] M. Mirbagheri, et al., Mater. Horiz., 6, 45-71 (2019)

[NPL 4] Kurosawa, H., Seibutsu Kagaku, Vol. 91, No.11, , p.646-653, 2013

## SUMMARY

[TECHNICAL PROBLEM]

Because large-volume culturing of adherent animal cells in a flat form such as on a culturing plate or dish requires large areas, layered-type culture apparatuses have been created for this purpose. Other methods using microcarriers for culturing of floatable supports on culture media have also been developed, and methods of culturing large amounts of adherent cells as suspended cells have also been constructed and implemented. Such methods have been applied to cause growth of cells for certain periods and for production of viruses or proteins, but no methodology has yet been discovered for achieving prolonged, stable culturing of cells without impairing the properties of the cells.

Mass culturing of animal cells also requires large amounts of oxygen to be continuously dissolved in the medium. Methods of dissolving oxygen in media include medium stirring methods and bubbling methods. However, most animal cells are susceptible to shearing force, resulting in the problem of cell death caused by stir culture methods. Even in common stir culture methods using a combination of a magnetic stirrer and stirring bar, the cells become mashed at the contact portion between the culture vessel and the stirring bar, hampering efforts to achieve mass culturing of the cells. Culturing methods that involve bubbling have also been problematic in that the cells are killed by foam generated during the bubbling. For mesenchymal stem cells which prefer to grow in low-oxygen environments it is undesirable to continuously dissolve large amounts of oxygen into the medium. Culturing of such cells requires additional equipment to lower the oxygen concentration in the incubator.

[SOLUTION TO PROBLEM]

The present inventors have reconsidered cell culture technology using polyimide porous membranes, and have created a static-type long-term mass cell culturing system that requires absolutely no special devices, by changing the conventional cell culturing porous membrane sheet size to a smaller size of about 1 mm per side. It was found that by forming small-piece porous membranes, it becomes possible to insert large amounts of sheets in very small spaces, and to thus supply nutrients and to accomplish mass culturing of cells in a stable and operational efficient manner. In addition, while it has been difficult to efficiently collect cells from polyimide porous membranes, it was demonstrated that the present method also allows cell collection to proceed with a certain degree of efficiency. Moreover, since using small pieces helped to provide satisfactory distribution of the medium in a dispersed state or layered state, the small-piece culturing method exhibited high efficiency for cell growth and exosome production volume. Since the property of the small pieces also improves the system visibility, it is possible to form a system with excellent productivity for a variety of substances including antibodies, viruses and exosomes. Sampling is also facilitated, and fixing, staining, identification and evaluation can be clearly carried out even with extraction of extremely small representative samples. It can also be used in combination with continuous culture methods that employ an overflow reactor, for example, allowing large amounts of cells to be cultured under static conditions for long periods. For exosome production in particular, it has been found that exosomes of high quality can be produced with stable production levels for long periods by a cell culturing method using the small-piece polymer porous membranes of the invention.

By using small-piece polymer porous membranes, the present invention can provide optimal space for mass culturing of cells without requiring special cell culturing equipment, and without using a stirrer in the cell culturing vessel or using a spinner flask. It was further found that during culturing, the small-piece polymer porous membranes dispersed in a culture solution and/or small-piece polymer porous membranes aggregated together in layers settle down to the bottom of the cell culturing vessel in their dispersed and/or layered state, and can maintain this state, thereby allowing the liquid volume (depth) of the cell culture solution to be easily adjusted and consequently allowing convenient adjustment of the amount of dissolved oxygen in the culture solution (the state of oxygen supply), and it was upon this finding that the present invention has been completed. Specifically, the present invention preferably includes, but is not limited to, the following aspects.

[1] A cell culturing method which comprises applying cells to small-piece polymer porous membranes and culturing them, and which does not require stirring, wherein:

the small-piece polymer porous membranes are small-piece polymer porous membranes with a three-layer structure comprising a surface layer A and a surface layer B having numerous pores, and a macro-void layer sandwiched between the surface layer A and surface layer B, the mean pore size of the pores in the surface layer A being smaller than the mean pore size of the pores in the surface layer B, the macro-void layer having partitions bonded to the surface layers A and B and numerous macro-voids surrounded by the partitions and the surface layers A and B, and the pores of the surface layers A and B communicating with the macro-voids, the area of the surface layer A or surface layer B is 4 mm$^2$ or smaller, and
the small-piece polymer porous membranes are dispersed and/or the small-piece polymer porous membranes are aggregated together in layers in the culture solution, and dispersed and/or layered at the bottom of the cell culturing vessel.

[2] The cell culturing method according to [1], wherein long term culturing is carried out by intermittent or continuous exchange of the medium.

[3] The cell culturing method according to [1] or [2], wherein subculturing and mass culturing of the cells is carried out by a process in which, after cells adhering to the small-piece polymer porous membranes have proliferated, the cells are detached by enzymatic treatment and small-piece polymer porous membranes which do not have adhered cells are added to the cell culturing vessel.

[4] The cell culturing method according to any one of [1] to [3], wherein the small-piece polymer porous membranes have a plurality of pores with mean pore sizes of 0.01 to 100 $\mu$m.

[5] The cell culturing method according to any one of [1] to [4], wherein the mean pore size of the surface layer A is 0.01 to 50 $\mu$m.

[6] The cell culturing method according to any one of [1] to [5], wherein the mean pore size of the surface layer B is 20 to 100 $\mu$m.

[7] The cell culturing method according to any one of [1] to [6], wherein the total film thickness of the small-piece polymer porous membranes is 5 to 500 $\mu$m.

[8] The cell culturing method according to any one of [1] to [7], wherein the small-piece polymer porous membranes are small-piece polyimide porous membranes.

[9] The cell culturing method according to [8], wherein the small-piece polyimide porous membranes are small-piece polyimide porous membranes comprising polyimide obtained from a tetracarboxylic dianhydride and a diamine.

[10] The cell culturing method according to [8] or [9], wherein the small-piece polyimide porous membranes are colored small-piece polyimide porous membranes obtained by molding a polyamic acid solution composition containing a polyamic acid solution obtained from a tetracarboxylic dianhydride and a diamine, and a coloring precursor, and then heat treating at 250°C or higher.

[11] The cell culturing method according to any one of [1] to [10], which includes producing exosomes from the cells.

[12] A cell culturing apparatus to be used in the cell culturing method according to any one of [1] to [11], which comprises small-piece polymer porous membranes.

[13] A kit to be used in the cell culturing method according to any one of [1] to [11], which comprises small-piece polymer porous membranes.

[14] Exosomes acquired by the method according to any one of [1] to [11].

[ADVANTAGEOUS EFFECTS OF INVENTION]

According to the present invention it is possible to carry out cell culturing with small-piece polymer porous membranes dispersed and/or small-piece polymer porous membranes aggregated together in layers in a culture solution, in a state where the small-piece polymer porous membranes are dispersed and/or layered at the bottom of the cell culturing vessel, so that the cell culture can be allowed to stand without requiring stirring with a stirrer or the like, and therefore, unlike use of a modularized polymer porous membrane comprising a conventional casing, it is not limited in terms of the size, material or strength of the cell culturing vessel and there is no need to install a stirrer or the like, thus allowing a very wide range of cell culturing vessels to be used. However, the cell culturing method of the invention merely obviates the need for stirring and does not exclude use under stirring conditions. It is therefore possible to employ light vibration or plastic flow conditions in the cell culturing method of the invention. In addition, after the cells have been seeded and cultured on the small-piece polymer porous membranes, additional small-piece polymer porous membranes can be added without collecting the cells, thereby allowing the amount of cells to be increased (internal expansion). Using the small-piece polymer porous membranes also allows stable and long-term substance production to be accomplished. Compared to a conventional polyimide porous membrane, culturing with the small-piece polymer porous membranes significantly increases cell proliferation and notably improves the cell recovery efficiency, while also allowing the aforementioned internal expansion and facilitating long-term, continuous cell culturing. Another advantage is that the small-piece members can be frozen and stored while the cells are supported in the small-piece polymer porous membranes.

BRIEF DESCRIPTION OF DRAWINGS

Fig. 1 is a graph showing time-dependent change in glucose consumption of human mesenchymal stem cells cultured using small-piece polyimide porous membranes (1 mm-square) and non-small-piece polyimide porous membranes (1 cm-square).

Fig. 2 is a graph showing long-term production of exosomes from human mesenchymal stem cells cultured using small-piece polyimide porous membranes.

Fig. 3 is a graph showing time-dependent change in the number of human mesenchymal stem cells.

Fig. 4 is a pair of photographs showing the results of stem cell verification for human mesenchymal stem cells cultured using small-piece polyimide porous membranes (left: induced differentiation of adipocytes (left panel); right: induced differentiation to osteoblasts and calcification).

Fig. 5 is a graph showing time-dependent change in the number of human skin fibroblasts cultured using small-piece polyimide porous membranes.

Fig. 6 is a diagram showing an example of an overflow-type bioreactor.

Fig. 7 is a diagram and photograph of an example of an overflow-type bioreactor.

Fig. 8 is a diagram showing an example of an overflow-type bioreactor.

Fig. 9 is a graph showing time-dependent change in cell behavior (cell density and cell behavior) of chondrocytes cultured using small-piece polyimide porous membranes.

DESCRIPTION OF EMBODIMENTS

Embodiments of the invention will now be explained with reference to the accompanying drawings as necessary. The constructions of the embodiments serve merely for example and the construction of the invention is not limited by the concrete constructions of the embodiments.

A. Small-piece polymer porous membranes

1. Fabrication of small-piece polymer porous membranes

The small-piece polymer porous membranes can be fabricated by shaping the polymer porous membrane described below into a predetermined size. The shaping may be carried out using any of a variety of processing units. As a non-limitative example, small-piece polymer porous membranes of the desired shapes can be obtained by punching a polymer porous membrane (for example, a polyimide porous membrane or polyethersulfone (PES) porous membrane) with a Pinnacle Die using a Pinnacle Simple Punch Unit.

2. Polymer porous membrane

An average pore diameter of the pore present on a surface layer A (hereinafter referred to as "surface A" or "mesh surface") in the polymer porous membrane used for the present invention is not particularly limited, but is, for example, 0.01 μm or more and less than 200 μm, 0.01 to 150 μm, 0.01 to 100 μm, 0.01 to 50 μm, 0.01 to 40 μm, 0.01 to 30 μm, 0.01 to 25 μm, 0.01 to 20 μm, or 0.01 to 15 μm, preferably 0.01 to 25 μm.

The average pore diameter of the pore present on a surface layer B (hereinafter referred to as "surface B" or "large pore surface") in the polymer porous membrane used for the present invention is not particularly limited so long as it is larger than the average pore diameter of the pore present on the surface A, but is, for example, greater than 5 μm and 200 μm or less, 20 μm to 100 μm, 25 μm to 100 μm, 30 μm to 100 μm, 35 μm to 100 μm, 40 μm to 100 μm, 50 μm to 100 μm, or 60 μm to 100 μm, preferably 30 μm to 100 μm.

In this specification, the average pore diameter on the surface of the polymer porous membrane is the area average pore diameter. The area average pore diameter can be determined according to the following (1) and (2). Incidentally, the average pore diameter of the portion other than the surface of the polymer porous membrane can be similarly determined.

(1) From the scanning electron micrograph of the surface of the porous membrane, the pore area S is measured for 200 or more open pore portions, and each pore diameter d is calculated from the following Equation I assuming the pore shape as a perfect circle.
[Math. 1]

$$\text{Pore Diameter d} = 2 \times \sqrt{(S/\pi)} \qquad \text{Equation I}$$

(2) All the pore diameters obtained by the above Equation I are applied to the following Equation II to determine the area average pore diameter da when the shape of the pores is a perfect circle.
[Math. 2]

$$\text{Area Average pore Diameter da} = \Sigma \, (d^3) \,/\, \Sigma \, (d^2) \qquad \text{Equation II}$$

The thicknesses of the surface layers A and B are not particularly limited, but is, for example, 0.01 to 50 $\mu$m, preferably 0.01 to 20 $\mu$m.

The average pore diameter of macrovoids in the planar direction of the membrane in the macrovoid layer in the polymer porous membrane is not particularly limited but is, for example, 10 to 500 $\mu$m, preferably 10 to 100 $\mu$m, and more preferably 10 to 80 $\mu$m. The thicknesses of the partition wall in the macrovoid layer are not particularly limited, but is, for example, 0.01 to 50 $\mu$m, preferably 0.01 to 20 $\mu$m. In an embodiment, at least one partition wall in the macrovoid layer has one or two or more pores connecting the neighboring macrovoids and having the average pore diameter of 0.01 to 100 $\mu$m, preferably 0.01 to 50 $\mu$m. In another embodiment, the partition wall in the macrovoid layer has no pore.

The total film thickness of the polymer porous membrane used for the invention is not particularly limited, but may be 5 $\mu$m or more, 10 $\mu$m or more, 20 $\mu$m or more or 25 $\mu$m or more, and 500 $\mu$m or less, 300 $\mu$m or less, 100 $\mu$m or less, 75 $\mu$m or less, or 50 $\mu$m or less. It is preferably 5 to 500 $\mu$m, and more preferably 25 to 75 $\mu$m.

The film thickness of the polymer porous membrane used for the invention can be measured using a contact thickness gauge.

The porosity of the polymer porous membrane used in the present invention is not particularly limited but is, for example, 40% or more and less than 95%.

The porosity of the polymer porous membrane used for the invention can be determined by measuring the film thickness and mass of the porous membrane cut out to a prescribed size, and performing calculation from the basis weight according to the following Equation III.
[Math. 3]

$$\text{Porosity} \% = (1 - w \,/\, (S \times d \times D)) \times 100 \quad \text{Equation III}$$

(wherein S represents the area of the porous membrane, d represents the total film thickness, w represents the measured mass, and D represents the polymer density. The density is defined as 1.34 g/cm$^3$ when the polymer is a polyimide.)

The polymer porous membrane used for the present invention is preferably a polymer porous membrane which includes a three-layer structure polymer porous membrane having a surface layer A and a surface layer B, the surface layers having a plurality of pores, and a macrovoid layer sandwiched between the surface layers A and B; wherein the average pore diameter of the pore present on the surface layer A is 0.01 $\mu$m to 25 $\mu$m, and the average pore diameter of the pore present on the surface layer B is 30 $\mu$m to 100 $\mu$m; wherein the macrovoid layer has a partition wall bonded to the surface layers A and B, and a plurality of macrovoids surrounded by such a partition wall and the surface layers A and B, the thickness of the macrovoid layer, and the surface layers A and B is 0.01 to 20 $\mu$m; wherein the pores on the surface layers A and B communicate with the macrovoid, the total film thickness is 5 to 500 $\mu$m, and the porosity is 40% or more and less than 95%. In an embodiment, at least one partition wall in the macrovoide layer has one or two or more pores connecting the neighboring macrovoids with each other and having the average pore diameter of 0.01 to 100 $\mu$m, preferably 0.01 to 50 $\mu$m. In another embodiment, the partition wall does not have such pores.

The polymer porous membrane used for the present invention is preferably sterilized. The sterilization treatment is not particularly limited, but any sterilization treatment such as dry heat sterilization, steam sterilization, sterilization with a disinfectant such as ethanol, electromagnetic wave sterilization such as ultraviolet rays or gamma rays, and the like can be mentioned.

The small-piece polymer porous membranes used for the invention are not particularly restricted so long as they have the aforementioned structural features, but they are preferably small-piece polyimide porous membranes or small-piece polyethersulfone (PES) porous membranes. The polyimide porous membrane and polyethersulfone (PES) porous membrane used to fabricate the small-piece polymer porous membranes will now be described.

2-1. Polyimide porous membrane

Polyimide is a general term for polymers containing imide bonds in the repeating unit, and usually it refers to an aromatic polyimide in which aromatic compounds are directly linked by imide bonds. An aromatic polyimide has an aromatic-aromatic conjugated structure via an imide bond, and therefore has a strong rigid molecular structure, and since the imide bonds provide powerful intermolecular force, it has very high levels of thermal, mechanical and chemical properties.

The polyimide porous membrane usable for the present invention is a polyimide porous membrane preferably containing polyimide (as a main component) obtained from tetracarboxylic dianhydride and diamine, more preferably a polyimide porous membrane composed of tetracarboxylic dianhydride and diamine. The phrase "including as the main component" means that it essentially contains no components other than the polyimide obtained from a tetracarboxylic dianhydride and a diamine, as constituent components of the polyimide porous membrane, or that it may contain them but they are additional components that do not affect the properties of the polyimide obtained from the tetracarboxylic dianhydride and diamine.

In an embodiment, the polyimide porous membrane usable for the present invention includes a colored polyimide porous membrane obtained by forming a polyamic acid solution composition including a polyamic acid solution obtained from a tetracarboxylic acid component and a diamine component, and a coloring precursor, and then heat treating it at 250°C or higher.

A polyamic acid is obtained by polymerization of a tetracarboxylic acid component and a diamine component. A polyamic acid is a polyimide precursor that can be cyclized to a polyimide by thermal imidization or chemical imidization.

The polyamic acid used may be any one that does not have an effect on the invention, even if a portion of the amic acid is imidized. Specifically, the polyamic acid may be partially thermally imidized or chemically imidized.

When the polyamic acid is to be thermally imidized, there may be added to the polyamic acid solution, if necessary, an imidization catalyst, an organic phosphorus-containing compound, or fine particles such as inorganic fine particles or organic fine particles. In addition, when the polyamic acid is to be chemically imidized, there may be added to the polyamic acid solution, if necessary, a chemical imidization agent, a dehydrating agent, or fine particles such as inorganic fine particles or organic fine particles. Even if such components are added to the polyamic acid solution, they are preferably added under conditions that do not cause precipitation of the coloring precursor.

In this specification, a "coloring precursor" is a precursor that generates a colored substance by partial or total carbonization under heat treatment at 250°C or higher.

Coloring precursors usable for the production of the polyimide porous membrane are preferably uniformly dissolved or dispersed in a polyamic acid solution or polyimide solution and subjected to thermal decomposition by heat treatment at 250°C or higher, preferably 260°C or higher, even more preferably 280°C or higher and more preferably 300°C or higher, and preferably heat treatment in the presence of oxygen such as air, at 250°C, preferably 260°C or higher, even more preferably 280°C or higher and more preferably 300°C or higher, for carbonization to produce a colored substance, more preferably producing a black colored substance, with carbon-based coloring precursors being most preferred.

The coloring precursor, when being heated, first appears as a carbonized compound, but compositionally it contains other elements in addition to carbon, and also includes layered structures, aromatic crosslinked structures and tetrahedron carbon-containing disordered structures.

Carbon-based coloring precursors are not particularly restricted, and for example, they include tar or pitch such as petroleum tar, petroleum pitch, coal tar and coal pitch, coke, polymers obtained from acrylonitrile-containing monomers, ferrocene compounds (ferrocene and ferrocene derivatives), and the like. Of these, polymers obtained from acrylonitrile-containing monomers and/or ferrocene compounds are preferred, with polyacrylonitrile being preferred as a polymer obtained from an acrylonitrile-containing monomer.

Moreover, in another embodiment, examples of the polyimide porous membrane which may be used for the preset invention also include polyimide porous membrane which can be obtained by molding a polyamic acid solution derived from a tetracarboxylic acid component and a diamine component followed by heat treatment without using the coloring precursor.

The polyimide porous membrane produced without using the coloring precursor may be produced, for example, by casting a polyamic acid solution into a film, the polyamic acid solution being composed of 3 to 60% by mass of polyamic acid having an intrinsic viscosity number of 1.0 to 3.0 and 40 to 97% by mass of an organic polar solvent, immersing or contacting in a coagulating solvent containing water as an essential component, and imidating the porous membrane of the polyamic acid by heat treatment. In this method, the coagulating solvent containing water as an essential component may be water, or a mixed solution containing 5% by mass or more and less than 100% by mass of water and more than 0% by mass and 95% by mass or less of an organic polar solvent. Further, after the imidation, one surface of the resulting polyimide porous membrane may be subjected to plasma treatment.

The tetracarboxylic dianhydride which may be used for the production of the polyimide porous membrane may be any tetracarboxylic dianhydride, selected as appropriate according to the properties desired. Specific examples of tetracarboxylic dianhydrides include biphenyltetracarboxylic dianhydrides such as pyromellitic dianhydride, 3,3',4,4'-biphenyltetracarboxylic dianhydride (s-BPDA) and 2,3,3',4'-biphenyltetracarboxylic dianhydride (a-BPDA), oxydiphthalic dianhydride, diphenylsulfone-3,4,3',4'-tetracarboxylic dianhydride, bis(3,4-dicarboxyphenyl)sulfide dianhydride, 2,2-bis(3,4-dicarboxyphenyl)-1,1,1,3,3,3-hexafluoropropane dianhydride, 2,3,3',4'-benzophenonetetracarboxylic dianhydride, 3,3',4,4'-benzophenonetetracarboxylic dianhydride, bis(3,4-dicarboxyphenyl)methane dianhydride, 2,2-bis(3,4-dicarboxyphenyl)propane dianhydride, p-phenylenebis(trimellitic acid monoester acid anhydride), p-biphenylenebis(trimellitic acid monoester acid anhydride), m-terphenyl-3,4,3',4'-tetracarboxylic dianhydride, p-terphenyl-3,4,3',4'-tetracarboxylic dianhydride, 1,3-bis(3,4-dicarboxyphenoxy)benzene dianhydride, 1,4-bis(3,4-dicarboxyphenoxy)benzene dianhydride, 1,4-bis(3,4-dicarboxyphenoxy)biphenyl dianhydride, 2,2-bis[(3,4-dicarboxyphenoxy)phenyl]propane dianhydride, 2,3,6,7-naphthalenetetracarboxylic dianhydride, 1,4,5,8-naphthalenetetracarboxylic dianhydride, 4,4'-(2,2-hexafluoroisopropylidene)diphthalic dianhydride, and the like. Also preferably used is an aromatic tetracarboxylic acid such as 2,3,3',4'-diphenylsulfonetetracarboxylic acid. These may be used alone or in appropriate combinations of two or more.

Particularly preferred among these are at least one type of aromatic tetracarboxylic dianhydride selected from the group consisting of biphenyltetracarboxylic dianhydride and pyromellitic dianhydride. As a biphenyltetracarboxylic dianhydride there may be suitably used 3,3',4,4'-biphenyltetracarboxylic dianhydride.

As diamine which may be used for the production of the polyimide porous membrane, any diamine may be used. Specific examples of diamines include the following.

1) Benzenediamines with one benzene nucleus, such as 1,4-diaminobenzene(paraphenylenediamine), 1,3-diaminobenzene, 2,4-diaminotoluene and 2,6-diaminotoluene;

2) diamines with two benzene nuclei, including diaminodiphenyl ethers such as 4,4'-diaminodiphenyl ether and 3,4'-diaminodiphenyl ether, and 4,4'-diaminodiphenylmethane, 3,3'-dimethyl-4,4'-diaminobiphenyl, 2,2'-dimethyl-4,4'-diaminobiphenyl, 2,2'-bis(trifluoromethyl)-4,4'-diaminobiphenyl, 3,3'-dimethyl-4,4'-diaminodiphenylmethane, 3,3'-dicarboxy-4,4'-diaminodiphenylmethane, 3,3',5,5'-tetramethyl-4,4'-diaminodiphenylmethane, bis(4-aminophenyl)sulfide, 4,4'-diaminobenzanilide, 3,3'-dichlorobenzidine, 3,3'-dimethylbenzidine, 2,2'-dimethylbenzidine, 3,3'-dimethoxybenzidine, 2,2'-dimethoxybenzidine, 3,3'-diaminodiphenyl ether, 3,4'-diaminodiphenyl ether, 4,4'-diaminodiphenyl ether, 3,3'-diaminodiphenyl sulfide, 3,4'-diaminodiphenyl sulfide, 4,4'-diaminodiphenyl sulfide, 3,3'-diaminodiphenylsulfone, 3,4'-diaminodiphenylsulfone, 4,4'-diaminodiphenylsulfone, 3,3'-diaminobenzophenone, 3,3'-diamino-4,4'-dichlorobenzophenone, 3,3'-diamino-4,4'-dimethoxybenzophenone, 3,3'-diaminodiphenylmethane, 3,4'-diaminodiphenylmethane, 4,4'-diaminodiphenylmethane, 2,2-bis(3-aminophenyl)propane, 2,2-bis(4-aminophenyl)propane, 2,2-bis(3-aminophenyl)-1,1,1,3,3,3-hexafluoropropane, 2,2-bis(4-aminophenyl)-1,1,1,3,3,3-hexafluoropropane, 3,3'-diaminodiphenyl sulfoxide, 3,4'-diaminodiphenyl sulfoxide and 4,4'-diaminodiphenyl sulfoxide;

3) diamines with three benzene nuclei, including 1,3-bis(3-aminophenyl)benzene, 1,3-bis(4-aminophenyl)benzene, 1,4-bis(3-aminophenyl)benzene, 1,4-bis(4-aminophenyl)benzene, 1,3-bis(4-aminophenoxy)benzene, 1,4-bis(3-aminophenoxy)benzene, 1,4-bis(4-aminophenoxy)benzene, 1,3-bis(3-aminophenoxy)-4-trifluoromethylbenzene, 3,3'-diamino-4-(4-phenyl)phenoxybenzophenone, 3,3'-diamino-4,4'-di(4-phenylphenoxy)benzophenone, 1,3-bis(3-aminophenyl sulfide)benzene, 1,3-bis(4-aminophenyl sulfide)benzene, 1,4-bis(4-aminophenyl sulfide)benzene, 1,3-bis(3-aminophenylsulfone)benzene, 1,3-bis(4-aminophenylsulfone)benzene, 1,4-bis(4-aminophenylsulfone)benzene, 1,3-bis[2-(4-aminophenyl)isopropyl]benzene, 1,4-bis[2-(3-aminophenyl)isopropyl]benzene and 1,4-bis[2-(4-aminophenyl)isopropyl]benzene;

4) diamines with four benzene nuclei, including 3,3'-bis(3-aminophenoxy)biphenyl, 3,3'-bis(4-aminophenoxy)biphenyl, 4,4'-bis(3-aminophenoxy)biphenyl, 4,4'-bis(4-aminophenoxy)biphenyl, bis[3-(3-aminophenoxy)phenyl]ether, bis[3-(4-aminophenoxy)phenyl]ether, bis[4-(3-aminophenoxy)phenyl]ether, bis[4-(4-aminophenoxy)phenyl]ether, bis[3-(3-aminophenoxy)phenyl]ketone, bis[3-(4-aminophenoxy)phenyl]ketone, bis[4-(3-aminophenoxy)phenyl]ketone, bis[4-(4-aminophenoxy)phenyl]ketone, bis[3-(3-aminophenoxy)phenyl] sulfide, bis[3-(4-aminophenoxy)phenyl] sulfide, bis[4-(3-aminophenoxy)phenyl] sulfide, bis[4-(4-aminophenoxy)phenyl] sulfide, bis[3-(3-aminophenoxy)phenyl]sulfone, bis[3-(4-aminophenoxy)phenyl]sulfone, bis[4-(3-aminophenoxy)phenyl]sulfone, bis[4-(4-aminophenoxy)phenyl]sulfone, bis[3-(3-aminophenoxy)phenyl]methane, bis[3-(4-aminophenoxy)phenyl]methane, bis[4-(3-aminophenoxy)phenyl]methane, bis[4-(4-aminophenoxy)phenyl]methane, 2,2-bis[3-(3-aminophenoxy)phenyl]propane, 2,2-bis[3-(4-aminophenoxy)phenyl]propane, 2,2-bis[4-(3-aminophenoxy)phenyl]propane, 2,2-bis[4-(4-aminophenoxy)phenyl]propane, 2,2-bis[3-(3-aminophenoxy)phenyl]-1,1,1,3,3,3-hexafluoropropane, 2,2-bis[3-(4-aminophenoxy)phenyl]-1,1,1,3,3,3-hexafluoropropane, 2,2-bis[4-(3-aminophenoxy)phenyl]-1,1,1,3,3,3-hexafluoropropane and 2,2-bis[4-(4-aminophenoxy)phenyl]-1,1,1,3,3,3-hexafluoropropane.

These may be used alone or in mixtures of two or more. The diamine used may be appropriately selected according to the properties desired.

Preferred among these are aromatic diamine compounds, with 3,3'-diaminodiphenyl ether, 3,4'-diaminodiphenyl ether, 4,4'-diaminodiphenyl ether, paraphenylenediamine, 1,3-bis(3-aminophenyl)benzene, 1,3-bis(4-aminophenyl)benzene, 1,4-bis(3-aminophenyl)benzene, 1,4-bis(4-aminophenyl)benzene, 1,3-bis(4-aminophenoxy)benzene and 1,4-bis(3-aminophenoxy)benzene being preferred for use. Particularly preferred is at least one type of diamine selected from the group consisting of benzenediamines, diaminodiphenyl ethers and bis(aminophenoxy)phenyl.

From the viewpoint of heat resistance and dimensional stability under high temperature, the polyimide porous membrane which may be used for the invention is preferably formed from a polyimide obtained by combination of a tetracarboxylic dianhydride and a diamine, having a glass transition temperature of 240°C or higher, or without a distinct transition point at 300°C or higher.

From the viewpoint of heat resistance and dimensional stability under high temperature, the polyimide porous membrane which may be used for the invention is preferably a polyimide porous membrane comprising one of the following aromatic polyimides.

(i) An aromatic polyimide comprising at least one tetracarboxylic acid unit selected from the group consisting of biphenyltetracarboxylic acid units and pyromellitic acid units, and an aromatic diamine unit,

(ii) an aromatic polyimide comprising a tetracarboxylic acid unit and at least one type of aromatic diamine unit selected from the group consisting of benzenediamine units, diaminodiphenyl ether units and bis(aminophenoxy)phenyl units, and/or,

(iii) an aromatic polyimide comprising at least one type of tetracarboxylic acid unit selected from the group consisting of biphenyltetracarboxylic acid units and pyromellitic acid units, and at least one type of aromatic diamine unit selected from the group consisting of benzenediamine units, diaminodiphenyl ether units and bis(aminophenoxy)phenyl units.

The polyimide porous membrane used in the present invention is preferably a three-layer structure polyimide porous membrane having a surface layer A and a surface layer B, the surface layers having a plurality of pores, and a macrovoid layer sandwiched between the surface layers A and B; wherein an average pore diameter of the pores present in the surface layer A is 0.01 $\mu$m to 25 $\mu$m, and the mean pore diameter present on the surface layer B is 30 $\mu$m to 100 $\mu$m; wherein the macrovoid layer has a partition wall bonded to the surface layers A and B, and a plurality of macrovoids surrounded by such a partition wall and the surface layers A and B; wherein the thickness of the macrovoid layer, and the surface layers A and B is 0.01 to 20 $\mu$m, wherein the pores on the surface layers A and B communicate with the macrovoid, the total film thickness is 5 to 500 $\mu$m, and the porosity is 40% or more and less than 95%. In this case, at least one partition wall in the macrovoid layer has one or two or more pores connecting the neighboring macrovoids and having the average pore diameter of 0.01 to 100 $\mu$m, preferably 0.01 to 50 $\mu$m.

For example, polyimide porous membranes described in WO2010/038873, Japanese Unexamined Patent Publication (Kokai) No. 2011-219585 or Japanese Unexamined Patent Publication (Kokai) No. 2011-219586 may be used for the present invention.

2-2. Polyethersulfone (PES) porous membrane

The polyethersulfone porous membrane which may be used for the present invention contains polyethersulfone and typically consists substantially of polyethersulfone. Polyethersulfone may be synthesized by the method known to those skilled in the art. For example, it may be produced by a method wherein a dihydric phenol, an alkaline metal compound and a dihalogenodiphenyl compound are subjected to polycondensation reaction in an organic polar solvent, a method wherein an alkaline metal di-salt of a dihydric phenol previously synthesized is subjected to polycondensation reaction dihalogenodiphenyl compound in an organic polar solvent or the like.

Examples of an alkaline metal compound include alkaline metal carbonate, alkaline metal hydroxide, alkaline metal hydride, alkaline metal alkoxide and the like. Particularly, sodium carbonate and potassium carbonate are preferred.

Examples of a dihydric phenol compound include hydroquinone, catechol, resorcin, 4,4'-biphenol, bis (hydroxyphenyl)alkanes (such as 2,2-bis(hydroxyphenyl)propane, and 2,2-bis(hydroxyphenyl)methane), dihydroxydiphenylsulfones, dihydroxydiphenyl ethers, or those mentioned above having at least one hydrogen on the benzene rings thereof substituted with a lower alkyl group such as a methyl group, an ethyl group, or a propyl group, or with a lower alkoxy group such as a methoxy group, or an ethoxy group. As the dihydric phenol compound, two or more of the aforementioned compounds may be mixed and used.

Polyethersulfone may be a commercially available product. Examples of a commercially available product include SUMIKAEXCEL 7600P, SUMIKAEXCEL 5900P (both manufactured by Sumitomo Chemical Company, Limited).

The logarithmic viscosity of the polyethersulfone is preferably 0.5 or more, more preferably 0.55 or more from the viewpoint of favorable formation of a macrovoid of the polyethersulfone porous membrane; and it is preferably 1.0 or less, more preferably 0.9 or less, further preferably 0.8 or less, particularly preferably 0.75 or less from the viewpoint of the easy production of a polyethersulfone porous membrane.

Further, from the viewpoints of heat resistance and dimensional stability under high temperature, it is preferred that the polyethersulfone porous membrane, or polyethersulfone as a raw material thereof has a glass transition temperature of 200°C or higher, or that a distinct glass transition temperature is not observed.

The method for producing the polyethersulfone porous membrane which may be used for the present invention is not particularly limited. For example, the membrane may be produced by a method including the following steps:

a step in which polyethersulfone solution containing 0.3 to 60% by mass of polyethersulfone having logarithmic viscosity of 0.5 to 1.0 and 40 to 99.7% by mass of an organic polar solvent is casted into a film, immersed in or contacted with a coagulating solvent containing a poor solvent or non-solvent of polyethersulfone to produce a coagulated film having pores; and
a step in which the coagulated film having pores obtained in the above-mentioned step is heat-treated for coarsening of the aforementioned pores to obtain a polyethersulfone porous membrane;
wherein the heat treatment includes the temperature of the coagulated film having the pores is raised higher than the glass transition temperature of the polyethersulfone, or up to 240°C or higher.

The polyethersulfone porous membrane which can be used in the present invention is preferably a polyethersulfone porous membrane having a surface layer A, a surface layer B, and a macrovoid layer sandwiched between the surface layers A and B,

wherein the macrovoid layer has a partition wall bonded to the surface layers A and B, and a plurality of macrovoids surrounded by such a partition wall and the surface layers A and B, the macrovoids having the average pore diameter in the planar direction of the membrane of 10 to 500 $\mu$m;
wherein the thickness of the macrovoid layer is 0.1 to 50 $\mu$m,
each of the surface layers A and B has a thickness of 0.1 to 50 $\mu$m,
wherein one of the surface layers A and B has a plurality of pores having the average pore diameter of more than 5 $\mu$m and 200 $\mu$m or less, while the other has a plurality of pores having the average pore diameter of 0.01 $\mu$m or more and less than 200 $\mu$m,
wherein one of the surface layers A and B has a surface aperture ratio of 15% or more while other has a surface aperture ratio of 10% or more,
wherein the pores of the surface layers A and B communicate with the macrovoids,
wherein the polyethersulfone porous membrane has total film thickness of 5 to 500 $\mu$m and a porosity of 50 to 95%.

Since the polymer porous membranes used as cell culture supports in the cell culturing apparatus of the invention have a slightly hydrophilic porous property, they retain liquid stably in the polymer porous membranes and maintain a moist environment that is also resistant to drying. It is therefore possible to achieve survival and proliferation of cells even in very small amounts of medium, compared to a cell culturing apparatus using a conventional cell culture support. Since cells seeded in the polymer porous membranes can be cultured without death of the cells by shearing force or foam, oxygen and nutrients can be efficiently supplied to the cells, allowing the cells to be mass-cultured.

The invention also allows sufficient oxygen to be supplied to cells.

2-3. Embodiment of small-piece polymer porous membranes to be used in cell culturing method

The small-piece polymer porous membranes to be used for an embodiment of the invention can be used by addition to any arbitrary cell culture solution, without housing a polymer porous membrane in a casing as in the prior art. According to one embodiment, the small-piece polymer porous membranes to be used in the cell culturing method of the invention are characterized in that the area of the surface layer A or surface layer B is 4 mm$^2$ or smaller, preferably 3 mm$^2$ or smaller, even more preferably 2 mm$^2$ or smaller and most preferably 1 mm$^2$. The lower limit for the area of the surface layer A or surface layer B may be 0.01 mm$^2$ or greater, 0.04 mm$^2$ or greater, 0.09 mm$^2$ or greater or 0.16 mm$^2$ or greater, for example. The shapes of the small-piece polymer porous membranes may be polygonal (such as triangular, quadrilateral (such as rectangular), pentagonal, hexagonal ... n-gonal (where n is any integer)), approximately circular, approximately elliptical, or shapes containing curves or lines. According to one embodiment, the longest diameter/shortest diameter ratio of surface layer A or surface layer B in the polymer porous membranes 200a applied in the cell culturing apparatus 1a is 0.5 to 1, preferably 0.75 to 1 and more preferably 0.9 to 1. As used herein, "diameter" means the length between two arbitrary points on the outer periphery of the surface layer A or surface layer B. The term "longest diameter" means the maximum length among the lengths between any two arbitrary points on the outer periphery of the surface layer A or surface layer B. The term "shortest diameter" means the minimum length among the lengths between any two arbitrary points on the outer periphery of the surface layer A or surface layer B. For this embodiment, when the small-piece polymer porous membranes used in the cell culturing method of the invention are quadrilateral, such as rectangular, the longest diameter may be $2 \times 2^{(1/2)}$ mm or smaller and the shortest diameter may be $2 \times 2^{(1/2)}$ mm or smaller, preferably the longest diameter is $1.5 \times 2^{(1/2)}$ mm and the shortest diameter is $1.5 \times 2^{(1/2)}$ mm or smaller, and more preferably the longest diameter is $1 \times 2^{(1/2)}$ mm or smaller and the shortest diameter is $1 \times 2^{(1/2)}$ mm or smaller. According to another mode, when the polymer porous membranes used in the cell culturing method of the invention are approximately circular or approximately elliptical, the longest diameter may be 2 mm or smaller and the shortest diameter may be 2 mm or smaller, preferably the longest diameter is 1.5 mm or smaller and the shortest diameter is 1.5 mm or smaller, and more preferably the longest diameter is 1.2 mm or smaller and the shortest diameter is 1.2 mm or smaller. This provides an effect of allowing sufficient supply of oxygen and nutrients to cells supported on the small-piece polymer porous membranes, resulting in satisfactory cell growth and significantly increasing the activity for production of substances such as proteins and exosomes. By adding the small-piece polymer porous membranes to medium in large amounts, the small-piece polymer porous membranes aggregate, and cells supported on any of the small-piece polymer porous membranes may be transferred to different small-piece polymer porous membranes, or they may be placed in a culturing environment causing them to grow while adhering to both. When the small-piece polymer porous membranes are applied to a cell culturing vessel, the cell culturing vessel is preferably used for static culturing without shaking. Since the small-piece polymer porous membranes settle in the medium when stationary, culturing of cells under low-oxygen conditions allows low-oxygen culturing to be conducted simply by adding medium to increase the depth, without using a special apparatus.

While the number of small-piece polymer porous membranes added to the cell culturing vessel is not particularly restricted, it is preferably a number such that the small-piece polymer porous membranes added to the culture solution are in a state aggregated together in layers. Each small-piece polymer porous membrane is preferably partially or fully overlapping with another small-piece polymer porous membrane, and specifically the overlapping portions may be 1%, 2%, 3%, 4%, 5%, 6%, 7%, 8%, 9%, 10%, 15%, 20%, 30%, 40%, 50%, 60%, 70%, 80%, 90%, 95%, 98%, 99% or 100%, of the small-piece polymer porous membranes.

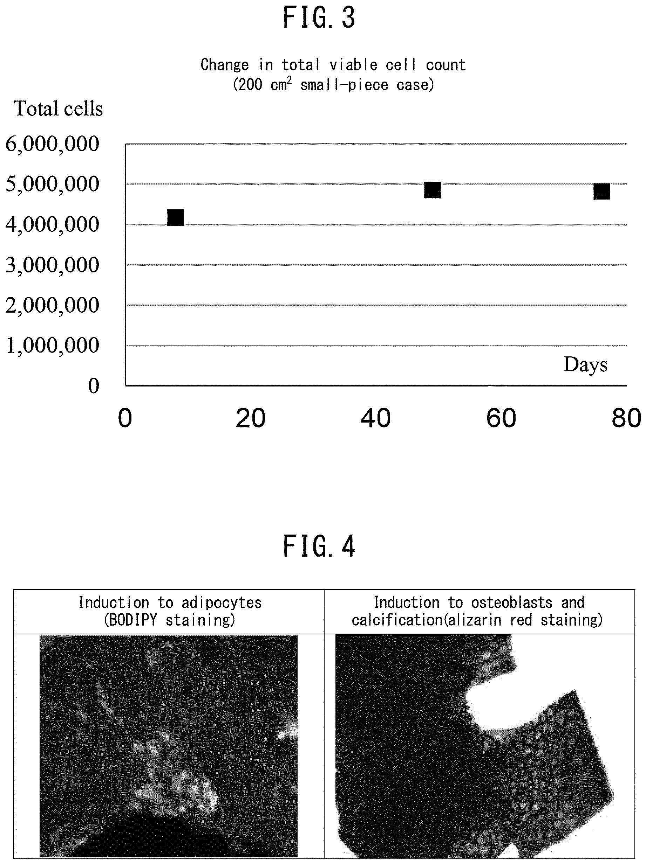

According to the invention, culturing using the small-piece polymer porous membranes may be with a regular multilayered structure as with a plurality of conventional polymer porous membranes stacked with the sides of the small-piece polymer porous membranes aligned in the vertical direction, but the small-piece polymer porous membranes may also be in an amorphous or non-uniform layered state. In the latter layered state, for example, using a simple example of two small-piece polymer porous membranes for explanation, one edge of one of the membranes may contact with one edge or side of the other membrane, forming a minute space in a V-shaped structure, a T-shaped structure or a lower-case y-shaped structure, instead of having a structure with both side faces of the membranes partially or fully overlapping. In contrast to this structure, during the course of their transfer or proliferation, seeded cells may aggregate not only on the flat sections of the membranes but also in the minute spaces formed between membranes, such as in the V-base portion in the case of a V-shaped structure, or at the mismatched levels between stacked surfaces (due to the membrane thicknesses or heights), while retaining a scaffolding in the small-piece polyimide porous membranes, or the cells may aggregate to create non-spheroidal three-dimensional spaces by the cells themselves, thus aiding their long-term survival. From the viewpoint of exosome production, it is possible to achieve stable production of exosomes with uniform quality, that is dependent on an environment with a stable and constant number of cells maintained in an aggregated state for long periods, instead of drastic variation due to proliferation and death. In an aggregated structure formed by the small-piece porous membranes and the cells growing on them, as can be seen in the photograph of Fig. 4 showing the results of verification of human mesenchymal stem cells in Example 2, adipocytes tend to aggregate and survive at the vertex of an inverted V-shaped structure between membranes (opposite the bottom of the V-shaped structure), while osteoblasts tend to form three-dimensional cell aggregates at mismatched level differences where membranes are layered, rather than at the flat sections of the membranes.

In a cell culturing method using microcarriers, as prior art, in addition to the issue of achieving high density culturing and cell growth, another issue is how to graft and seed cells onto the microcarriers. It is well-known that stirring and diffusion means are necessary to prevent microcarriers from clumping together, i.e. to prevent formation of aggregates, and this is considered to be a negative aspect in cell culturing. According to the invention, however, since small-piece polymer porous membranes are used for cell culturing and may be in a dispersed state and aggregated together, the problem of aggregate formation is not a problem when using the small-piece polymer porous membranes.

In addition, with small-piece polymer porous membranes that are dispersed or aggregated together in layers at the bottom of the cell culturing vessel in the culture solution, i.e. whose settling has stopped, because of the light weight of the small pieces themselves, they can remain in a floating state in the flow of the medium, for example, even though a vertex, edge or side of one small piece may contact with the bottom or side wall of the cell culturing vessel, or with a vertex, edge or side of another small piece. According to the invention, the small-piece polymer porous membranes are three-dimensionally dispersed and aggregated together in layers while floating and the polymer porous membranes are also liquid-permeable, and therefore cells supported on the small-piece polymer porous membranes are not depleted of nutrients or oxygen from the medium (the circulation is excellent), thus allowing culturing of the cells to be carried out in a stationary manner without shaking.

In previous culturing of cells where the polymer porous membrane continuously deforms and is not in a form of small pieces, it is known that cells growing in the polymer porous membrane are subjected to stress and often die due to apoptosis (WO2018/021367). It has been described that cell death is induced to a notable extent when readily deformable membrane strips of 0.5 cm length $\times$ 5 cm width (1:10 aspect ratio) are used as polymer porous membranes. Even if the small-piece polymer porous membranes of the invention are fabricated with the same aspect ratio as such polymer porous membranes, it will be readily appreciated that since the thicknesses of the small-piece polymer porous membranes are the same as the thickness of the polymer porous membrane mentioned above, the small-piece polymer porous membranes are more highly rigid in terms of their three-dimensional shapes, and have lower deformation efficiency. This also applies to small-piece polymer porous membranes of different forms in addition to membrane strips. For example, as demonstrated in Comparative Example 1 described below, when using "1 cm-square polyimide porous membranes" and " 1 mm-square small-piece polyimide porous membranes" for culturing of human mesenchymal stem cells, the use of small-piece polymer porous membranes resulted in higher glucose consumption throughout the culturing period, and therefore the cell culturing method using small-piece polymer porous membranes of the invention may be said to provide an environment more conducive to cell life than the cell culturing method with a conventional polymer porous membrane, from the viewpoint of cell growth, substance production and long-term culturing.

B. Cell culturing vessel

According to the invention, the cell culturing method of the invention is a method in which suspended cells are poured onto small-piece polymer porous membranes and cell culturing is carried out in a stationary manner, and it does not require application of a stirrer in the cell culturing vessel or the use of a spinner flask. As mentioned above, the small-piece polymer porous membranes have a property of dispersing and/or being mutually aggregated together in layers while settling at the bottom of the cell culturing vessel, and the cell culturing vessel and cell culturing apparatus are not particularly restricted so long as they allow the membranes to be in such a state in the medium. Hence, there is no limitation to a dish, petri dish, plate, well, bottle or bag as is commonly used for cell culturing, and the material is also not limited to plastic or glass, for example, so that any desired vessel may be used. When a cell culturing apparatus is used, the cell culturing vessel may be in the form of a culturing tank installed in the apparatus.

Since the small-piece polymer porous membranes have the property of being dispersed and/or mutually aggregated together in layers and settling at the bottom of the cell culturing vessel, and do not require stirring, long-term cell culturing is possible by using a cell culturing apparatus that comprises means for intermittent or continuous exchange of the medium. Examples for the cell culturing apparatus include, but are not limited to, an overflow-type bioreactor or an intermittent-type bioreactor (WO2018/021359).

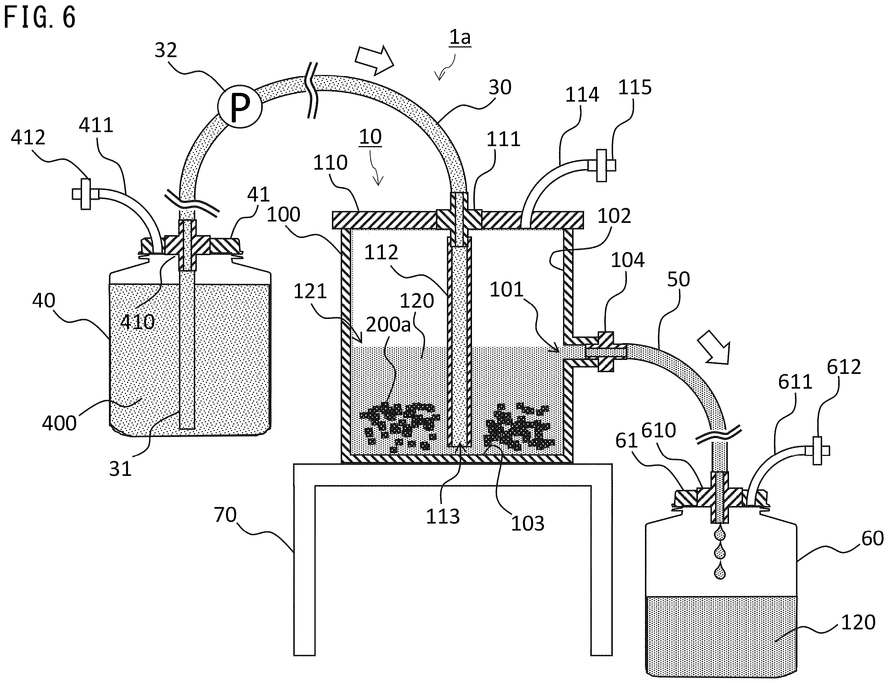

An overflow-type bioreactor is shown in Fig. 6 as an exemplary cell culturing apparatus. Specifically, the overflow-type bioreactor is a cell culturing apparatus 1a as shown in Fig. 6, comprising:

small-piece polymer porous membranes 200a onto which cells are applied;
a culturing tank 10 that houses the small-piece polymer porous membranes 200a;
a culture medium supply port 113 that supplies medium, provided in the culturing tank 10;
a culture medium discharge port 101 that discharges the medium, provided at a side section of the culturing tank 10;
a medium supply tank 40 in communication with the culture medium supply port 113 and provided outside the culturing tank 10; and
a medium recovery tank 60 which is in communication with the culture medium discharge port 101 and recovers the medium discharged from the culture medium discharge port 101; wherein the culture medium discharge port 101 discharges the medium in an overflow manner.

Using such a cell culturing apparatus, it is possible to continuously supply fresh culture medium, while also allowing the medium to be continuously recovered from the culture medium discharge port provided in the culturing tank, depending on the amount of medium supplied, so that long-term cell culturing can be achieved. Moreover, it is possible to prevent rapid changes in the concentration of proteins necessary for cell growth, which have been produced by the cells, as occurs with conventional medium exchange, and to continuously supply nutrients (such as glucose) in the medium that have been consumed by culturing, allowing the desired cell culture environment to be maintained.

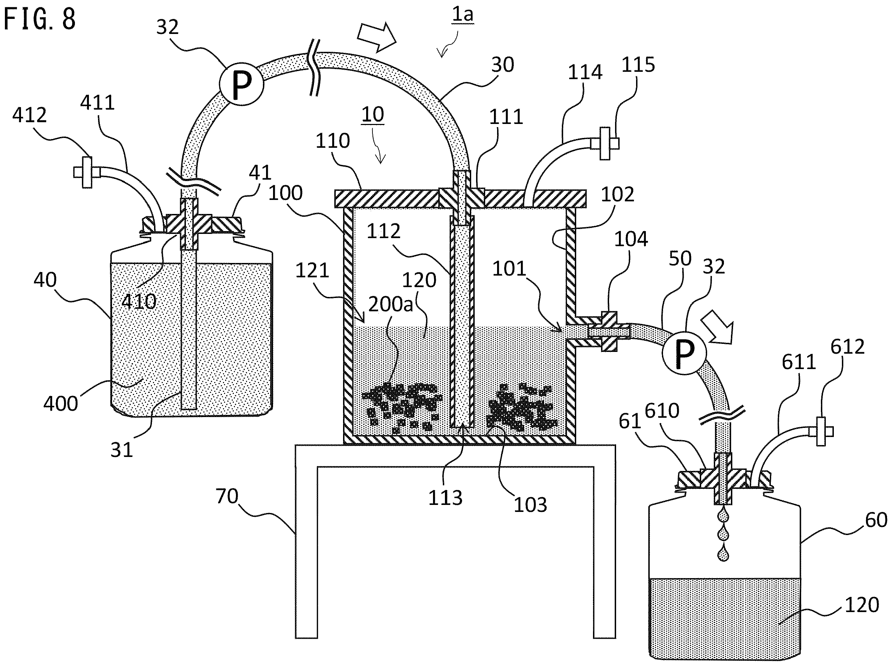

In the exemplary overflow-type bioreactor (Fig. 6), medium is discharged as overflow from the culture medium discharge port 101, but a pump 32 is also provided in a medium discharge tube 50 that is connected for recovery of the medium discharged from an exit port connector 104 into the medium recovery tank 60, thereby allowing the medium to be forcibly recovered after cell culturing (see Fig. 8).

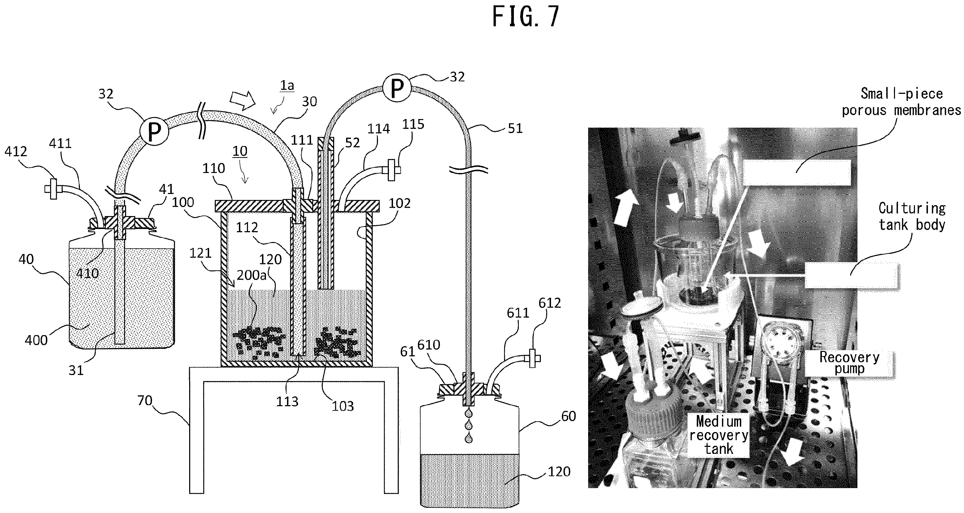

In another embodiment, an overflow-type bioreactor (Fig. 7) is provided which comprises a medium discharge tube 51 (for example, a tube having an inner diameter of 1 mm and an outer diameter of 2 mm) running into the interior at the top surface of the cell culture solution, through a supporting guide 52, instead of providing a culture medium discharge port 101 on the side 102 of the culturing tank main body 100 as shown in Fig. 6 and Fig. 8. In this bioreactor, liquid medium may be removed from the top after cell culturing to adjust the height of the medium.

C. Cell culturing method using small-piece polymer porous membranes

In the cell culturing method of the invention, cells are applied to and cultured on small-piece polymer porous membranes, without requiring agitation by stirring or the like. In addition, fresh culture medium can be supplied and the medium can be easily recovered after culturing without recovering the cells themselves or the small-piece polymer porous membranes supporting the cells, thus allowing long-term culturing to be carried out in a stationary manner. As used herein, "medium" refers to cell culture medium for culturing of cells, and especially animal cells. The term "medium" is used synonymously with the term "cell culture solution". Medium, for the purpose of the invention, is therefore liquid medium. The type of medium may be commonly employed medium, which may be appropriately determined for the type of cells to be cultured.

According to the invention there is no particular restriction on the specific process used for applying the cells to the small-piece polymer porous membranes. It is possible to carry out the steps described throughout the present specification, or to employ any desired method suited for applying cells onto small-piece polymer porous membranes. Application of cells to the small-piece polymer porous membranes in the method of the invention includes, but is not limited to, the following modes.

(A) Injection of cell suspension onto the small-piece polymer porous membranes that have been added to the cell culturing vessel;
(B) Addition of small-piece polymer porous membranes to the cell suspension that has been injected into the cell culturing vessel.

Cells seeded onto the surfaces of the polymer porous membranes adhere to the small-piece polymer porous membranes and infiltrate into the pores. Preferably, the cells adhere to the small-piece polymer porous membranes without applying any particular exterior physical or chemical force. The cells that have been seeded on the surfaces of the small-piece polymer porous membranes can stably grow and proliferate on the surfaces and/or in the interiors of the membranes. The cells may be in a variety of different forms, depending on the location of the membranes used for growth and proliferation. The small-piece polymer porous membranes used may also be wetted beforehand with cell culture solution or a sterilized liquid.

Preferably, but not restrictively, the viable cells are selectively retained in the small-piece polymer porous membranes. According to a preferred embodiment of the method of the invention, therefore, viable cells are retained in the small-piece polymer porous membranes while dead cells are left in the cell suspension, the dead cells being removed by flow of the medium or by exchange of the medium.

The sterilized liquid is not particularly restricted, and may be a sterilized buffering solution or sterilized water. A buffering solution may be, for example, (+) or (-) Dulbecco's PBS, or (+) or (-) Hank's Balanced Salt Solution. Examples of buffering solutions are listed in Table 1 below.

[Table 1]

| Component | Concentration (mmol/L) | Concentration (g/L) |
| --- | --- | --- |
| NaCl | 137 | 8.00 |
| KCl | 2.7 | 0.20 |
| $Na_2HPO_4$ | 10 | 1.44 |
| $KH_2PO_4$ | 1.76 | 0.24 |
| pH(-) | 7.4 | 7.4 |

In the method of the invention, application of cells to the small-piece polymer porous membranes further includes a mode of adding adherent cells in a floating state as a suspension together with the small-piece polymer porous membranes, to adhere the cells with the membranes (entangling). When the cell culture medium is liquid, the polymer porous membranes may be floating within the cell culture medium, but the cell-adhering small-piece polymer porous membranes rapidly sink in the medium and become aggregated together in layers, settling down to the bottom of the cell culturing vessel and being maintained in that state.

Furthermore, since the height of the medium in the cell culturing vessel housing the small-piece polymer porous membranes (for example, the culturing tank of the cell culturing apparatus) (i.e. the medium level) can be easily adjusted, the medium level can be raised to increase the culturing efficiency of mesenchymal stem cells, for example, that prefer a low-oxygen environment.

Since the cell culturing method of the invention does not require active agitation of the medium with a stirrer or the like, and the small-piece polymer porous membranes can settle to the bottom of the cell culturing vessel in a state dispersed and/or aggregated together in layers, it becomes possible to continuously supply fresh culture medium in a culturing system using an overflow-type bioreactor, for example, while continuously recovering medium from the culture medium discharge port provided in the culturing tank after cell culturing, depending on the amount of supplied medium, so that long-term cell culturing can be carried out. This also applies when using an intermittent-type bioreactor, as fresh culture medium can be supplied at predetermined time intervals and medium can be periodically recovered after cell culturing, allowing long-term cell culturing to be carried out.

Furthermore, by adding fresh, non-cell-supporting small-piece polymer porous membranes to the cell culturing vessel during continuous cell culturing, the cells can be transferred and adhered to the freshly added small-piece polymer porous membranes, allowing further growth (expansion culturing).

By thus using small-piece polymer porous membranes in the cell culturing method of the invention, it is possible to achieve satisfactory medium circulation and mass-culturing of cells. It is also possible to increase production of desired substances (such as proteins and exosomes). Human mesenchymal stem cells proliferated well when seeded on small-piece polymer porous membranes, as demonstrated in Example 1, and when the cells were cultured for long periods, exosomes were stably produced for long periods, as demonstrated in Example 2.

D. Kit for use in cell culturing method

The present invention further provides a kit to be used in a cell culturing method, the kit comprising small-piece polymer porous membranes. The kit of the invention may include constituent elements necessary for cell culturing in addition to the small-piece polymer porous membranes, as appropriate. This includes, for example, the cells applied to the small-piece polymer porous membranes, the cell culture medium and the instruction manual for the cell culturing apparatus and the kit. While not restrictive, one mode includes a package containing either one or a plurality of sterilized small-piece polymer porous membranes stored in a transparent pouch, in a form allowing their use for cell culturing, or a kit having a sterile liquid encapsulated together with small-piece polymer porous membranes in the same pouch, in the form of an integrated film/liquid allowing efficient suction seeding.

Definition of terms

As used herein, the term "suspended cells" refers to cells obtained by forcibly causing adherent cells to float and be suspended in the medium using proteases such as trypsin, for example, and adherent cells that have been rendered suitable for suspension culturing in medium by a known conditioning process.

The types of cells to be used for the invention may be selected from the group consisting of animal cells, insect cells, plant cells, yeast cells and bacteria. Animal cells are largely divided into cells from animals belonging to the subphylum Vertebrata, and cells from non-vertebrates (animals other than animals belonging to the subphylum Vertebrata). There are no particular restrictions on the source of the animal cells, for the purpose of the present specification. Preferably, they are cells from an animal belonging to the subphylum Vertebrata. The subphylum Vertebrata includes the superclass Agnatha and the superclass Gnathostomata, the superclass Gnathostomata including the class Mammalia, the class Aves, the class Amphibia and the class Reptilia. Preferably, they are cells from an animal belonging to the class Mammalia, generally known as mammals. Mammals are not particularly restricted but include, preferably, mice, rats, humans, monkeys, pigs, dogs, sheep and goats.

The types of animal cells that may be used for the invention are not particularly restricted, but are preferably selected from the group consisting of pluripotent stem cells, tissue stem cells, somatic cells and germ cells.

Throughout the present specification, the term "pluripotent stem cells" is intended as a comprehensive term for stem cells having the ability to differentiate into cells of a variety of tissues (pluripotent differentiating power). While not a restriction, pluripotent stem cells include embryonic stem cells (ES cells), induced pluripotent stem cells (iPS cells), embryonic germ cells (EG cells) and germ stem cells (GS cells). They are preferably ES cells or iPS cells. Particularly preferred are iPS cells, which are free of ethical problems, for example. Any publicly known pluripotent stem cells may be used, such as the pluripotent stem cells described in International Patent Publication No. 2009/123349(PCT/JP2009/057041).

The term "tissue stem cells" refers to stem cells that are cell lines capable of differentiation but only to limited specific tissues, though having the ability to differentiate into a variety of cell types (pluripotent differentiating power). For example, hematopoietic stem cells in the bone marrow are the source of blood cells, while neural stem cells differentiate into neurons. Additional types include hepatic stem cells from which the liver is formed and skin stem cells that form skin tissue. Preferably, the tissue stem cells are selected from among mesenchymal stem cells, hepatic stem cells, pancreatic stem cells, neural stem cells, skin stem cells and hematopoietic stem cells.

The term "somatic cells" refers to cells other than germ cells, among the cells composing a multicellular organism. In sexual reproduction these are not passed on to the next generation. Preferably, the somatic cells are selected from among hepatocytes, pancreatic cells, muscle cells, bone cells, osteoblasts, osteoclasts, chondrocytes, adipocytes, skin cells, fibroblasts, pancreatic cells, renal cells and lung cells, or blood cells such as lymphocytes, erythrocytes, leukocytes, monocytes, macrophages or megakaryocytes.

The term "germ cells" refers to cells having the role of passing on genetic information to the succeeding generation in reproduction. These include, for example, gametes for sexual reproduction, i.e. the ova, egg cells, sperms, sperm cells, and spores for asexual reproduction.

The cells may also be selected from the group consisting of sarcoma cells, established cell lines and transformants. The term "sarcoma" refers to cancer occurring in non-epithelial cell-derived connective tissue cells, such as the bone, cartilage, fat, muscle or blood, and includes soft tissue sarcomas, malignant bone tumors and the like. Sarcoma cells are cells derived from sarcoma. The term "established cell line" refers to cultured cells that are maintained *in vitro* for long periods and reach a stabilized character and can be semi-permanently subcultured. Existing cell lines include those derived from various tissues of various species including humans, such as PC12 cells (from rat adrenal medulla), CHO cells (from Chinese hamster ovary), HEK293 cells (from human embryonic kidney), HL-60 cells from (human leukocytes) and HeLa cells (from human cervical cancer), Vero cells (from African green monkey kidney epithelial cells), MDCK cells (from canine kidney epithelial cells), HepG2 cells (human hepatic cancer-derived cell line), BHK cells (baby hamster kidney cells) and NIH3T3 cells (from mouse fetal fibroblasts). The term "transformants" refers to cells with an altered genetic nature created by extracellularly introduced nucleic acid (DNA and the like).

As used herein, "adherent cells" generally refers to cells that must be adhered to appropriate surfaces for growth, such as adherent cells or scaffold-dependent cells. According to an embodiment of the invention, the cells used are adherent cells. The cells to be used for the invention are adherent cells and more preferably cells that can be cultured while suspended in medium. Adherent cells that can be suspension cultured can be obtained by conditioning adherent cells while in a state suitable for suspension culture using a publicly known method, and examples include CHO cells, HEK293 cells, Vero cells, NIH3T3 cells, and cell lines derived from such cells.

In the cell culturing method of the invention, application of cells and culturing are carried out on small-piece polymer porous membranes, thereby allowing convenient culturing of large volumes of cells to be accomplished since large numbers of cells grow on the multisided connected pore sections on the insides and on the surfaces of the small-piece polymer porous membranes. Cells seeded onto the small-piece polymer porous membranes, when used according to the invention, provide an environment which allows growth even under agitated conditions (which have caused cell death in the prior art), and consequently the cells can be cultured in large amounts.

Throughout the present specification, the volume of small-piece polymer porous membranes without cells, occupying space including the volume between the interior gaps, will be referred to as the "apparent small-piece polymer porous membrane volume". Where cells are applied to the small-piece polymer porous membranes, with the cells being supported on the surfaces and interiors of the small-piece polymer porous membranes, the volume of space occupied by the entirety of the small-piece polymer porous membranes, cells and medium infiltrating inside the small-piece polymer porous membranes will be referred to as the "small-piece polymer porous membrane volume including the cell-viable region". When the film thickness of the small-piece polymer porous membranes is 25 $\mu$m, the small-piece polymer porous membrane volume including the cell-viable region is a large value of, at maximum, about 50% greater than the apparent small-piece polymer porous membrane volume. In the method of the invention, a plurality of small-piece polymer porous membranes (at least 2, such as 3, 4, 5, 10, 20, 30, 40, 50, 100, 500, $1 \times 10^3$, $2 \times 10^3$, $3 \times 10^3$, $4 \times 10^3$, $5 \times 10^3$, $7 \times 10^3$, $1 \times 10^4$, $2 \times 10^4$, $3 \times 10^4$, $5 \times 10^4$, $7 \times 10^4$, $1 \times 10^5$, $2 \times 10^5$, $3 \times 10^5$, $5 \times 10^5$, $7 \times 10^5$, $1 \times 10^6$, $2 \times 10^6$, $3 \times 10^6$, $5 \times 10^6$, $7 \times 10^6$, $1 \times 10^7$ or greater), may be housed in a single cell culturing vessel for culturing, in which case the total sum of the small-piece polymer porous membrane volume including the cell-viable region of each of the plurality of cell-supporting small-piece polymer porous membranes may be referred to simply as the "small-piece polymer porous membrane volume including the cell-viable region".

Using the method of the invention, cells can be satisfactorily cultured for prolonged periods even under conditions in which the total volume of the cell culture medium in the cell culturing vessel is up to 10,000 times the total sum of the small-piece polymer porous membrane volume including the cell-viable region. Moreover, cells can be satisfactorily cultured for prolonged periods even under conditions in which the total volume of the cell culture medium in the cell culturing vessel is up to 1000 times the total sum of the small-piece polymer porous membrane volume including the cell-viable region. The cells can also be satisfactorily cultured for prolonged periods even under conditions in which the total volume of the cell culture medium in the cell culturing vessel is up to 100 times the total sum of the small-piece polymer porous membrane volume including the cell-viable region. Moreover, cells can be satisfactorily cultured for prolonged periods even under conditions in which the total volume of the cell culture medium in the cell culturing vessel is up to 10 times the total sum of the small-piece polymer porous membrane volume including the cell-viable region.

In other words, according to the invention the space (vessel) used for cell culturing can be reduced to an absolute minimum, compared to a cell culturing apparatus in which conventional two-dimensional culturing is carried out. When it is desired to increase the number of cells cultured, the cell culturing volume can be flexibly increased by a convenient procedure including increasing the number of layered small-piece polymer porous membranes. In a cell culturing apparatus comprising small-piece polymer porous membranes to be used for the invention, the space (vessel) in which cells are cultured and the space (vessel) in which the cell culture medium is stored can be separate, and the necessary amount of cell culture medium can be prepared according to the number of cells to be cultured. The space (vessel) in which the cell culture medium is stored can be increased or decreased according to the purpose, or it may be a replaceable vessel, with no particular restrictions.

As used herein, "mass culturing of cells" refers to culturing in which, for example, the number of cells in the cell culturing vessel after culturing using the small-piece polymer porous membranes reaches $1.0 \times 10^5$ or more, $1.0 \times 10^6$ or more, $2.0 \times 10^6$ or more, $5.0 \times 10^6$ or more, $1.0 \times 10^7$ or more, $2.0 \times 10^7$ or more, $5.0 \times 10^7$ or more, $1.0 \times 10^8$ or more, $2.0 \times 10^8$ or more, $5.0 \times 10^8$ or more, $1.0 \times 10^9$ or more, $2.0 \times 10^9$ or more, or $5.0 \times 10^9$ or more, per milliliter of medium, with the cells evenly dispersed in the cell culture medium in the cell culturing vessel.

The method used for counting the number of cells during or after culturing may be any of the known methods. For example, any publicly known method may be used to count the number of cells in the cell culturing vessel after culturing using the small-piece polymer porous membranes, when the cells are evenly dispersed in the cell culture medium in the cell culturing vessel. For example, a method of cell counting with a CCK8 may be used. Specifically, a Cell Counting Kit 8, as a solution reagent by Dojindo Laboratories (hereunder referred to as "CCK8") may be used to count the number of cells in ordinary culturing without using small-piece polymer porous membranes, and the correlation coefficient between the absorbance and the actual cell count determined. After then applying the cells, the cultured small-piece polymer porous membranes may be transferred to CCK8-containing medium and stored in an incubator for 1 to 3 hours, and then the supernatant collected and its absorbance measured at a wavelength of 460 nm, and the cell count determined from the previously calculated correlation coefficient.

From a different viewpoint, "mass culturing" of cells refers to culturing in which, for example, the number of cells per square millimeter of the small-piece polymer porous membranes after culturing using the small-piece polymer porous membranes reaches $1.0 \times 10^3$ or more, $2.0 \times 10^3$ or more, $1.0 \times 10^4$ or more, $2.0 \times 10^4$ or more, $5.0 \times 10^4$ or more, $1.0 \times 10^5$ or more, $2.0 \times 10^5$ or more, $5.0 \times 10^5$ or more, $1.0 \times 10^6$ or more, $2.0 \times 10^6$ or more or $5.0 \times 10^6$ or more. The number of cells per square centimeter of small-piece polymer porous membranes can be appropriately measured using a publicly known method, such as with a CCK8 as mentioned above.

All of the publications mentioned throughout the present specification are incorporated herein in their entirety by reference.

The examples of the invention described below are intended to serve merely as illustration and do not limit the technical scope of the invention. The technical scope of the invention is limited solely by the description in the Claims. Modifications of the invention, such as additions, deletions or substitutions to the constituent features of the invention, are possible so long as the gist of the invention is maintained.

EXAMPLES

The present invention will now be explained in greater detail by examples. It is to be understood, however, that the invention is not limited to these examples. A person skilled in the art may easily implement modifications and changes to the invention based on the description in the present specification, and these are also encompassed within the technical scope of the invention.

The polyimide porous membranes used to fabricate the small-piece polymer porous membranes in the Examples described below were prepared by molding a polyamic acid solution composition comprising a polyamic acid solution obtained from 3,3', 4,4'-biphenyltetracarboxylic dianhydride(s-BPDA) as a tetracarboxylic acid component and 4,4'-diaminodiphenyl ether (ODA) as a diamine component, and polyacrylamide as a coloring precursor, and then heat treating it at 250°C or higher. The obtained polyimide porous membrane was a polyimide porous membrane with a three-layer structure comprising a surface layer A and a surface layer B having numerous pores, and a macro-void layer sandwiched between the surface layer A and surface layer B, the mean pore size of the pores in surface layer A being 19 $\mu$m, the mean pore size of the pores in surface layer B being 42 $\mu$m, the film thickness being 25 $\mu$m and the porosity being 74%.

Example 1: Human mesenchymal stem cell culturing method and exosome production using small-piece polymer porous membranes

(1) Fabrication of small-piece polyimide porous membranes

A 1 mm × 1 mm flexible Pinnacle Die, AP type flat puncher by Tsukatani Hamono Mfg. Co., Ltd. (blade specification listed below) was prepared and fixed onto a magnet plate by magnetic force, and then a Simple Punch Unit (RDC FB type) by Ube Industries, Ltd. was used to punch out a polyimide porous membrane (25 $\mu$m thickness) to prepare 1 mm × 1 mm small-piece polyimide porous membranes.
Blade specification:

Blade height: 0.8 mm
Blade depth: 0.4 mm
Etching depth: 0.6 mm
Base thickness: 0.2 mm
Blade angle: 40°

(2) Preparation of mesenchymal stem cells and seeding onto small-piece polyimide porous membranes

Human mesenchymal stem cells (Poietics™) by Lonza Group, Ltd. were subcultured twice in a type 1 collagen-coated dish by Iwaki, and the cells (4.0 × 10^6 cells) were trypsin-treated and suspended in medium (ADSC-4 Xeno-Free medium by Kohjin Bio, 4 ml). The cell culture was poured onto small-piece polyimide porous membranes (200 cm^2) that had been aseptically wetted beforehand with 50 ml of the same medium in a 150 ml sterilization bottle by Corning, Inc. and shaken and suspended for 30 minutes in an incubator. After pouring and shaking several times, they were stationed in a $CO_2$ incubator and culturing was initiated.

(3) Continuous culturing

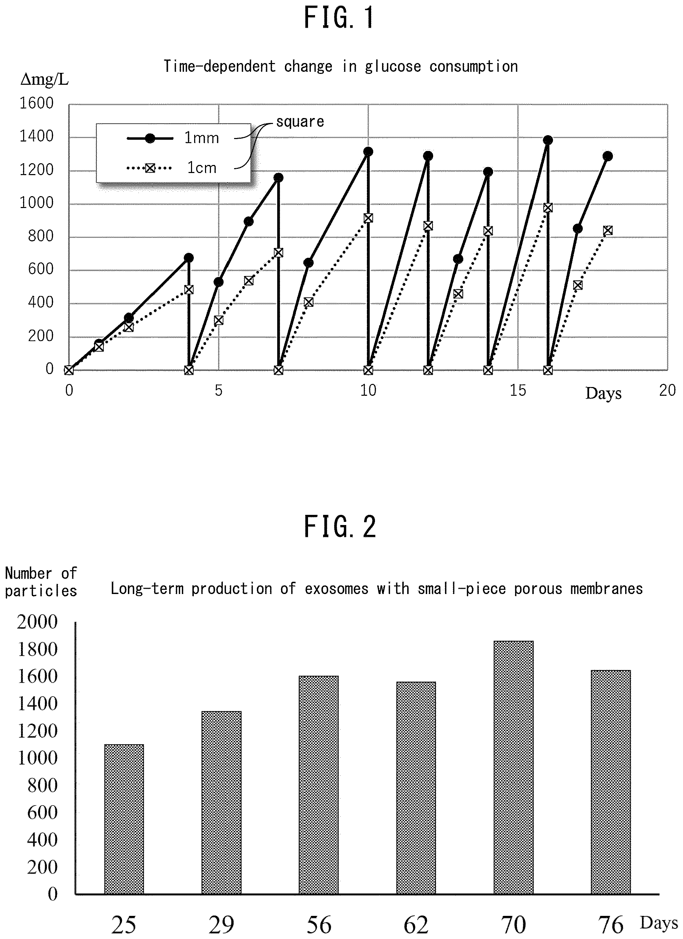

The medium (ADSC-4) was exchanged with 50 ml of medium containing 1000 mg/L glucose once every 2 or 3 days, and culturing was continued for 18 days. During the culture process, the glucose consumption and lactic acid production were continuously measured to confirm satisfactory cell growth (Fig. 1).

(4) Confirming cell count on small-piece polyimide porous membranes and recovery of cells from small-piece porous membranes

Color reaction with a Cell Counting Kit-8 by Dojindo Laboratories was used to verify the number of cells growing on the small-piece polyimide porous membranes 18 days after culturing was initiated, the result being a total grown count of 4.6 × 10^6 cells. After pouring 25 ml of TrypLE™ by Thermo Fisher Scientific onto the cell-grown sheet and allowing it to stand in the incubator for 50 minutes, the suspension was recovered and the small pieces were further rinsed with 10 ml of TrypLE™ to recover the cells. The recovered cell count was 2.3 × 10^6 cells (cell yield: 50%). The recovered cells could be cultured on a collagen-coated dish.

(5) Measurement of number of exosomes

The recovered cell culture solution was centrifuged using a high-speed refrigerated centrifuge (Model 6000 by Kubota Corp.) at 4°C, 10,000 g for 30 minutes to remove the debris (preparation solution 1). This "preparation solution 1" was diluted to a predetermined concentration using PBS (-) (product of FujiFilm-Wako, distributor code: 166-23555) with the microparticles removed by suction filtration using a 0.025 $\mu$m filter (product name: MF-Millipore membrane filter by Merck, Ltd., Model: VSWP04700) (preparation solution 2). The particles of 200 nm and larger were then removed from "preparation solution 2" using a 0.2 $\mu$m syringe filter (product name: MiniSart by Sartorius, Japan, Model: 16534K) (preparation solution 3). A zeta potential/particle size distribution meter (ZEECOM ZC-3000 by Microtec-Nition Co., Ltd.) was used to measure the particle size distribution and the number of particles in the exosome fraction in "preparation solution 3", based on Brownian motion trajectory analysis. The amount of exosome production per day was calculated by the following "Formula 1".
Formula 1: Number of particles × sample dilution factor × proportion of particles of 150 nm or smaller/number of days embedded in medium

Example 2

(1) Long-term culturing using small-piece polyimide porous membranes

Long-term culturing of human mesenchymal stem cells was carried out by the same method as Example 1, and the medium was periodically recovered to obtain the exosomes. Stabilized exosomes were obtainable throughout the culturing period (Fig. 2). The total cell count at 76 days after the start of culturing was $4.82 \times 10^6$ cells. Fig. 3 shows the change in cell count during the experiment period.

(2) Verification of stem cells on small-piece polyimide porous membranes

In order to verify cultured human mesenchymal stem cells on the small-piece polyimide porous membranes, culture samples in different periods were partially recovered and were induced to differentiate to adipocytes or osteoblasts. The results are shown in Fig. 4. This confirmed that stem cell potential was maintained throughout the culturing period.

Comparative Example 1: Method of culturing human mesenchymal stem cells using polymer porous membranes

(1) Culturing using 1 cm-square polyimide porous membranes.

Instead of the 1 mm-square small-piece polyimide porous membranes described in Example 1, 200 1 cm-square polyimide porous membranes were prepared and seeded to the same cell density as in the experiment of Example 1, as a comparative experiment. The total cultured cell count after 18 days was $3.0 \times 10^6$ cells.

(2) Recovery of mesenchymal stem cells from 1 cm-square polyimide porous membranes

In a cell recovery experiment using TrypLE™ in the same manner as Example 1, the recovered free cell count was $1.0 \times 10^6$ cells (cell yield: 33%).

(3) Metabolism-based comparison

The comparative data for glucose consumption in Example 1 and Comparative Example 1 are shown in Fig. 1. It was confirmed that high glucose consumption was maintained by culturing on the small-piece polyimide porous membranes throughout the culturing period.

Example 3: Method of culturing human skin fibroblasts using small-piece polyimide porous membranes

(1) Preparation and cell seeding of skin fibroblasts.

Human adult skin fibroblasts that had been cultured on a 60 cm$^2$ dish were detached and recovered using trypsin, and 3 ml of FibroLife Xeno-Free medium by Lifeline Co. was added to prepare a cell suspension ($3.0 \times 10^6$ cells). The cell culture was poured onto small-piece polyimide porous membranes (300 cm$^2$) that had been aseptically wetted beforehand with 25 ml of the same medium and shaken and suspended for 30 minutes in an incubator. After pouring and shaking several times, they were stationed in a CO$_2$ incubator and culturing was initiated.

(2) Culturing of skin fibroblasts on small-piece polyimide porous membranes

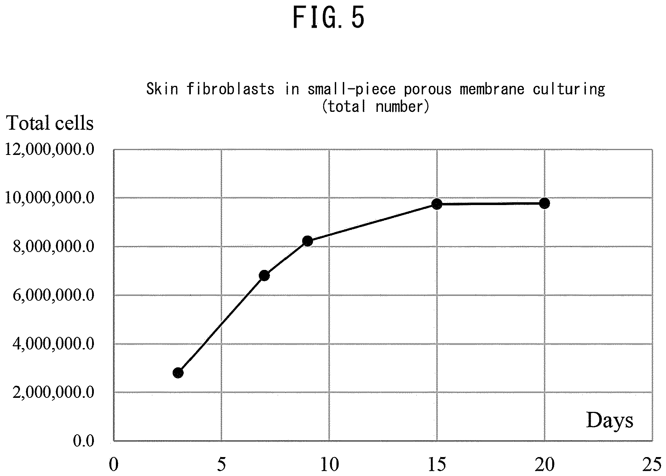

The medium was exchanged every 2 or 3 days, with daily medium exchange from 15 days onward. Fig. 5 shows the change in total cell count based on coloring using a CCK8. Stable cell growth was observed.

Example 4: Intermittent culturing of hybridomas using small-piece polyimide porous membranes (1) Preparation of hybridomas and seeding onto small-piece porous membranes

A JCR B cell bank hybridoma (SC78.H81.C81.A9) was subcultured 8 times with a cell culture dish (Falcon™) by Corning and a 125 mL Erlenmeyer flask by Thermo Fisher Scientific (subculturing 4 times with medium containing 10% ES Cell FBS by Gibco™ added to RPMI-1640 by FujiFilm-Wako, followed by subculturing 4 times with medium containing 8 mM GlutaMax™ by Thermo Fisher Scientific added to serum-free medium CD Hybridoma by Thermo Fisher Scientific), to prepare a cell suspension with a viable cell density of $1.17 \times 10^6$ cells/mL (viable cell rate: 87%). After aseptically transferring 120 cm$^2$ γ-ray sterilized $1 \times 1$ mm small-piece porous membranes wetted with purified water into a 125 mL Erlenmeyer flask by Thermo Fisher Scientific (Model 4115-0125), 10 mL of the aforementioned medium (8 mM GlutaMax™-added CD Hybridoma) was poured over them and shaking was carried out for 30 minutes in a $CO_2$ incubator to complete wetting of the membranes. The medium was then removed by discharge, thus completing preparation of the wetted membranes in the Erlenmeyer flask. After pouring 20 mL of cell suspension into the flask and shaking several times, it was allowed to stand for 1 day in a $CO_2$ incubator to complete adsorption of the cells onto the small-piece porous membranes. The total amount of residual solution in the flask was removed by discharge, and after pouring in 20 mL of medium (8 mM GlutaMax™-added CD Hybridoma), it was stationed in a $CO_2$ incubator and culturing was initiated.

(2) Measurement of metabolites and antibody production

A small amount of cell culture solution was sampled on the 2nd day and 6th day after the start of culturing, and a CedexBio by Roche Corp. was used to measure the glucose concentration, lactic acid concentration and antibody production in the culture solution, to verify the state of metabolism. Satisfactory cell grafting and proliferation were confirmed (Table 2), and progression to efficient antibody production was also confirmed. This indicated that the hybridomas were able to be cultured on the small-piece porous membranes and were suitable for continuous antibody production.

[Table 2]

| Culturing period (days) | GLC (mg/L) | LAC (mg/L) | MIgG (mg/L) |
|---|---|---|---|
| 2 | 3357 | 126 | 4.76 |
| 6 | 2162 | 1497 | 27.53 |

(3) Measurement of cell count on small-piece porous membranes

After rinsing the small-piece porous membranes twice with 10 mL of Ham's F-12 medium (containing glutamine and phenol red) by FujiFilm-Wako on the 6th day of culturing to remove the suspended cells, the viable cell density adhering to the small-piece porous membranes was measured by color reaction using a Cell Counting Kit-8 by Dojindo Laboratories, and a number of $1.97 \times 10^5$ viable cells were observed at a cell density of $1.64 \times 10^3$ cells/cm$^2$. The cells that did not easily detach by rinsing were confirmed to be adhering and grafted onto the small-piece porous membranes.

Example 5: Intermittent culturing of hybridomas using small-piece polyimide porous membranes

(1) Preparation of hybridomas and seeding onto small-piece porous membranes

Cells and small-piece porous membranes prepared by the same method as Example 1 were poured into a culturing tank and shaken for 24 hours at 50 rpm in a $CO_2$ incubator using a shaker (MaxQ 200 CO2Plus) by Thermo Fisher Scientific, after which they were transferred to stationary culture. After transfer they were cultured by the same procedure as in Example 4.

(2) Measurement of metabolites and antibody production

Samples were obtained by the same method as Example 4 and measured for metabolites and antibody production, thereby confirming grafting and satisfactory proliferation of the cells. Excellent antibody production ability was confirmed (Table 3). More efficient small-piece porous membrane culturing was accomplished when the cells were adhered using mobile conditions, thus confirming their suitability for efficient antibody production.

[Table 3]

| Culturing period (days) | GLC (mg/L) | LAC (mg/L) | MIgG (mg/L) |
|---|---|---|---|
| 2 | 3305 | 192 | 5.38 |
| 6 | 1682 | 1972 | 43.43 |

(3) Measurement of cell count on small-piece porous membranes

When the viable cell density adhering to the small-piece porous membranes was measured by the same method as Example 4, a total viable cell count of $1.70 \times 10^5$ cells was observed at a cell density of $1.41 \times 10^3$ cells/cm$^2$. The hybridomas were confirmed to be adhering and grafted onto the small-piece porous membranes, in a state that was not easily detachable even with rinsing several times.

Example 6: Hybridomas in continuous culturing using small-piece polyimide porous membranes (1) Preparation of hybridomas and seeding onto small-piece porous membranes

A JCR B cell bank hybridoma (SC78.H81.C81.A9) was cultured by suspension culture with subculturing 10 times with a cell culture dish (Falcon™) by Corning and a 125 mL Erlenmeyer flask by Thermo Fisher Scientific (subculturing 4 times with 10% FBS-added RPMI-1640 medium, followed by subculturing 6 times with 8 mM GlutaMax™-added CD Hybridoma medium), to prepare a cell suspension with a viable cell density of $1.11 \times 10^6$ cells/mL (viable cell rate: 78%). A 20 mL portion of the cell suspension was poured into an overflow reactor as shown in Fig. 7, and filled with 120 cm$^2$ of sterilized $1 \times 1$ mm small-piece porous membranes that had been wetted with purified water. After pouring and shaking the culturing tank portion several times, it was stationed in a $CO_2$ incubator for 30 minutes and culturing was initiated. The apparatus shown in Fig. 8 was also usable in the same manner.

(2) Continuous culturing

The aforementioned medium (8 mM GlutaMax™-added CD Hybridoma) was continuously supplied into a reactor at about 20 mL per day, and an extraction pump (tube pump by As One Corp.) was used to control the liquid level height for continuous recovery of the medium while maintaining a constant medium level in the culturing tank.
It is possible to maintain a stable culturing environment with a fixed amount of medium in the tank by controlling to the same liquid level, using a discharge pump on the right side as in the drawing when using the apparatus of Fig. 8, or by natural overflow without a pump (Fig. 6).

(3) Measurement of metabolites

The extracted culture solution was recovered once per day, and the glucose concentration, lactic acid concentration and antibody production level in the recovered liquid were measured using a CedexBio by Roche Corp. The results are shown in Table 4. The results confirm that stable and efficient glucose consumption and lactic acid production proceeded throughout the culturing period, while satisfactory antibody production was also stably and continuously achieved.

[Table 4]

| Culturing period (days) | Recovered liquid volume | GLC (mg/L) | LAC (mg/L) | MIgG (mg/L) |
|---|---|---|---|---|
| 1 | 25 | 2029 | 1365 | 26.10 |
| 2 | 12 | 2338 | 1240 | 21.99 |
| 3 | 24 | 2748 | 918 | 16.05 |
| 4 | 15 | 2683 | 995 | 17.92 |
| 5 | 15 | 2565 | 1126 | 21.04 |

(4) Measurement of cell count on small-piece porous membranes

The medium in the reactor was removed on the 5th day of culturing, and the remaining small-piece porous membranes were rinsed twice with 10 mL of Ham's F-12 medium (containing glutamine and phenol red) by FujiFilm-Wako, removing the suspended cells that were detachable from the surfaces, and then the cell count was measured by color reaction using a Cell Counting Kit-8 by Dojindo Laboratories. The cell density was measured by this method to be $1.46 \times 10^3$ cells/cm$^2$, and a total count of viable cells of $1.75 \times 10^5$ cells was confirmed to be adhered and grafted onto the small-piece porous membranes in a state that was not easily detachable even by rinsing.

Example 7: Cell culturing and protein production by skin fibroblasts on small-piece polyimide porous membranes

(1) Small-piece porous membrane preparation and cell culturing

Human skin fibroblasts (Lot No. 18TL215675 by Lonza) were subcultured 5 times in a FALCON cell culture dish by Corning and trypsin-treated, and the cells ($1.2 \times 10^6$ cells) were suspended in medium (1.2 ml FGM-2 BulletKit by Lonza Group, Ltd.). In a 125 ml sterilized bottle by Corning, Inc., the D-PBS was removed from small-piece polyimide porous membranes (120 cm$^2$) that had been aseptically wetted beforehand in a $CO_2$ incubator with 50 ml of D-PBS (FujiFilm-Wako), and then 10 ml of medium (FGM-2 BulletKit, Lonza Group, Ltd.) was poured in. After allowing the porous membrane-containing medium to stand in the same incubator for another 30 minutes, 1.2 ml of cell suspension was poured in. After pouring and shaking several times, the mixture was allowed to stand overnight in the same incubator, and on the next day, 20 ml of the medium (FGM-2 BulletKit by Lonza Group, Ltd.) was poured in to initiate long-term culturing.

(2) Continuous culturing

Continuous culturing was carried out for 18 days with exchange of 30 ml of the medium (FGM-2 BulletKit) once per week. During the culture process, the glucose consumption and lactic acid production were measured to confirm satisfactory cell growth.

(3) Measurement of fibronectin production

The recovered cell culture solution was used for measurement of the fibronectin production levels on the 5th day, 13th day and 18th day, using a Fibronectin ELISA kit (Cat: MK115 by Takara). The results are shown in Table 5.

[Table 5]

| Culturing period (days) | Cell density (cells/cm$^2$) | Fibronectin production (ng/ml/day) |
|---|---|---|
| 5 | $3.0 \times 10^4$ | 526 |
| 13 | $6.4 \times 10^4$ | 747 |
| 18 | $7.1 \times 10^4$ | 1008 |

Example 8: Small-piece porous membrane culturing of chondrocytes

A 125 ml rectilinear PET storage bottle (45 mm cap) by Corning was prepared containing 120 cm$^2$ of sterilized small-piece polyimide porous membranes (1 mm $\times$ 1 mm). A glucose solution (45 w/v% D (+)-glucose solution by FujiFilm-Wako) was poured into medium (KBM ADSC-4R Xeno-Free medium by Kohjin Bio) in a rectilinear sterilized bottle containing the small-piece porous membranes, and 30 ml of medium adjusted to a total glucose concentration of 3000 mg/L was poured in and the small pieces were thoroughly mingled with the medium.

Human chondrocytes (Y30 female_439Z037.3) by PromoCell that had been subcultured 4 times in a collagen I-coated dish by Iwaki were detached using Trypsin-EDTA (0.05%) and phenol red by Gibco, subjected to centrifugation and cell recovery procedures, and then suspended in the aforementioned prepared medium (glucose-added KBM ADSC-4R). A cell suspension with $1.0 \times 10^4$ cells per 1 cm$^2$ of area of the small-piece polyimide porous membranes (total: $1.2 \times 10^6$ cells) was poured in at a suspended cell density of $1.0 \times 10^6$ cells per 1 ml. After pouring, the square bottle of the culturing tank containing the small-piece porous membranes was transferred to a $CO_2$ incubator and stationary culture was initiated.

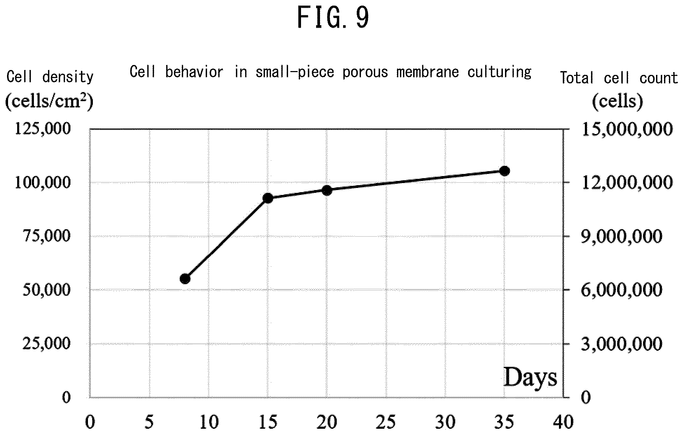

Continuous culturing was carried out with exchange of the medium in an amount of 30 ml twice a week. The total number of cells growing on the small-piece porous membranes was measured at 8 days, 15 days, 20 days and 35 days using color reaction with a Cell Counting Kit-8 by Dojindo Laboratories. The state of proliferation is shown in Fig. 9. A satisfactory state of cell growth of the human chondrocytes was observed. This demonstrated that large amounts of cells can be conveniently and easily cultured for long periods by stable, long-term culturing by stationary culture in a small culture vessel.

When the production level of exosomes in the recovered medium was measured on the 19th day of culturing using a CD9/CD63 ELISA kit for human exosome quantitation (Model _EXH0102EL, product of Cosmo Bio Co., Ltd.), in terms of the amount of CD63 protein, 117 pg/ml of exosomes were confirmed to be produced by accumulation within 3 days. Efficient and stable exosome production was also confirmed in an intermittent culturing system, similar to the continuous culture system described above.

Example 9: Small-piece porous membrane culturing and cell recovery of human skin fibroblasts (1) Small-piece porous membrane preparation and cell preparation

Sterilized small-piece polyimide porous membranes (300 cm$^2$) were aseptically transferred into a 50 ml centrifuge tube by Sumitomo Bakelite Co., Ltd. (screw cap conical tube), and then 12 ml of PBS was poured in to wet the membranes. After one hour, the PBS was removed by suction to complete preparation of the membranes.

Human skin fibroblasts (Lot No. 18TL215675 by Lonza) were subcultured twice in a FALCON cell culture dish by Corning and trypsin-treated, and then a glucose solution was added to the medium (FibroLife$^R$) Fibroblast Serum Free Medium Complete Kit by Lifeline), and after adjustment to 2000 mg/L, the cells ($3.0 \times 10^6$ cells) were suspended in 3.0 ml of the medium.

(2) Cell seeding and cell culturing

After adding 10 ml of medium and the aforementioned cell suspension to previously prepared small-piece porous membranes in the conical tube, and gently shaking several times, the mixture was allowed to stand overnight in a $CO_2$ incubator. On the following day, the cell-adsorbed small-piece polyimide porous membranes were transferred into a 150 ml sterilized bottle by Corning, Inc., 20 ml of medium was further added, and culturing was initiated. The medium (30 ml volume) was exchanged twice a week, and the total number of cells growing on the small-piece porous membranes was measured at 2 days, 6 days, 9 days and 14 days using color reaction with a Cell Counting Kit-8 by Dojindo Laboratories. On each measuring day, the cell density per 1 cm$^2$ of human skin fibroblasts growing on the small-piece polyimide porous membranes in the bottle and the total number of cells in the bottle were measured, to confirm the state of cell growth. The results are shown in Table 6.

[Table 6]

| Day | 2 | 6 | 9 | 14 |
|---|---|---|---|---|
| Cell density (cells/cm$^2$) | $7.7 \times 10^3$ | $1.4 \times 10^4$ | $2.5 \times 10^4$ | $2.4 \times 10^4$ |
| Total cell count (cells) | $2.3 \times 10^6$ | $4.3 \times 10^6$ | $7.4 \times 10^6$ | $7.3 \times 10^6$ |

(3) Cell recovery from small-piece porous membranes and subculturing

On the 15th day after the start of culturing, the medium in the bottle was eliminated and the small-piece porous membranes in the bottle were rinsed with 20 ml of PBS, after which the PBS was removed by suction. Next, 15 ml of TrypLE Express™ by Thermo Fisher Scientific was poured in, and the mixture was allowed to stand for 40 minutes in a $CO_2$ incubator. The supernatant that clouded after freeing of the cells was collected, the small-piece porous membranes were rinsed with 25 ml of medium, and the free cells were recovered. After centrifugal separation, they were suspended in 3 ml of medium, and the cell count was measured, confirming a count of $3.6 \times 10^6$ viable cells. The recovered cells were seeded onto a culture plate and onto small-piece polyimide porous membranes, and growth of the cells was confirmed.

REFERENCE SIGNS LIST

    1a Cell culturing apparatus
    10 Culturing tank
    100 Culturing tank body
    101 Culture medium discharge port
    102 Side section
    103 Bottom part
    104 Exit port connector
    110 Culturing tank cover
    111 First cover connector
    112 First medium supply tube
    113 Culture medium supply port

114 First vent tube
115 First ventilation filter
120 Cultured medium
121 Medium level
200a Small-piece polymer porous membranes 30 Second medium supply tube
31 Third medium supply tube
32 Pump
40 Medium supply tank
400 Fresh medium
41 Medium supply tank cover
410 Second cover connector
411 Second vent tube
412 Second ventilation filter
50, 51 Medium discharge tube
52 Guide
60 Medium recovery tank
61 Medium recovery tank cover
610 Third cover connector
611 Third vent tube
612 Third ventilation filter
70 Culturing tank platform

**Claims**

1. A cell culturing method which comprises applying cells to small-piece polymer porous membranes and culturing them, and which does not require stirring, wherein:

   the small-piece polymer porous membranes are small-piece polymer porous membranes with a three-layer structure comprising a surface layer A and a surface layer B having numerous pores, and a macro-void layer sandwiched between the surface layer A and surface layer B, the mean pore size of the pores in the surface layer A being smaller than the mean pore size of the pores in the surface layer B, the macro-void layer having partitions bonded to the surface layers A and B and numerous macro-voids surrounded by the partitions and the surface layers A and B, and the pores of the surface layers A and B communicating with the macro-voids, the area of the surface layer A or surface layer B is 4 mm$^2$ or smaller, and
   the small-piece polymer porous membranes are dispersed and/or the small-piece polymer porous membranes are aggregated together in layers in the culture solution, and dispersed and/or layered at the bottom of the cell culturing vessel.

2. The cell culturing method according to claim 1, wherein long-term culturing is carried out by intermittent or continuous exchange of the medium.

3. The cell culturing method according to claim 1 or 2, wherein subculturing and mass culturing of the cells is carried out by a process in which, after cells adhering to the small-piece polymer porous membranes have proliferated, the cells are detached by enzymatic treatment and small-piece polymer porous membranes which do not have adhered cells are added to the cell culturing vessel.

4. The cell culturing method according to any one of claims 1 to 3, wherein the small-piece polymer porous membranes have a plurality of pores with mean pore sizes of 0.01 to 100 $\mu$m.

5. The cell culturing method according to any one of claims 1 to 4, wherein the mean pore size of the surface layer A is 0.01 to 50 $\mu$m.

6. The cell culturing method according to any one of claims 1 to 5, wherein the mean pore size of the surface layer B is 20 to 100 $\mu$m.

7. The cell culturing method according to any one of claims 1 to 6, wherein the total film thickness of the small-piece polymer porous membranes is 5 to 500 $\mu$m.

8. The cell culturing method according to any one of claims 1 to 7, wherein the small-piece polymer porous membranes are small-piece polyimide porous membranes.

9. The cell culturing method according to claim 8, wherein the small-piece polyimide porous membranes are small-piece polyimide porous membranes comprising polyimide obtained from a tetracarboxylic dianhydride and a diamine.

10. The cell culturing method according to claim 8 or 9, wherein the small-piece polyimide porous membranes are colored small-piece polyimide porous membranes obtained by molding a polyamic acid solution composition containing a polyamic acid solution obtained from a tetracarboxylic dianhydride and a diamine, and a coloring precursor, and then heat treating it at 250°C or higher.

11. The cell culturing method according to any one of claims 1 to 10, which includes producing exosomes from the cells.

12. A cell culturing apparatus to be used in the cell culturing method according to any one of claims 1 to 11, which comprises small-piece polymer porous membranes.

13. A kit to be used in the cell culturing method according to any one of claims 1 to 11, which comprises small-piece polymer porous membranes.

14. Exosomes acquired by the method according to any one of claims 1 to 11.

# FIG. 1

Time-dependent change in glucose consumption

# FIG. 2

Long-term production of exosomes with small-piece porous membranes

# FIG. 3

Change in total viable cell count
(200 cm$^2$ small-piece case)

# FIG. 4

| Induction to adipocytes (BODIPY staining) | Induction to osteoblasts and calcification(alizarin red staining) |
| --- | --- |
| | |

# FIG. 5

Skin fibroblasts in small-piece porous membrane culturing
(total number)

FIG. 6

FIG. 7

Small-piece porous membranes

Culturing tank body

Recovery pump

Medium recovery tank

FIG. 8

EP 4 012 016 A1

# FIG. 9

Cell behavior in small-piece porous membrane culturing

Cell density (cells/cm$^2$)

Total cell count (cells)

Days

## INTERNATIONAL SEARCH REPORT

International application No.

PCT/JP2020/030614

### A. CLASSIFICATION OF SUBJECT MATTER

C12M 3/00(2006.01)i; C12N 5/00(2006.01)i; C12N 5/0775(2010.01)n; C12N 5/20(2006.01)n
FI: C12N5/00; C12M3/00 A; C12N5/0775; C12N5/20

According to International Patent Classification (IPC) or to both national classification and IPC

### B. FIELDS SEARCHED

Minimum documentation searched (classification system followed by classification symbols)
C12M3/00; C12N5/00; C12N5/0775; C12N5/20

Documentation searched other than minimum documentation to the extent that such documents are included in the fields searched

| | |
|---|---|
| Published examined utility model applications of Japan | 1922-1996 |
| Published unexamined utility model applications of Japan | 1971-2020 |
| Registered utility model specifications of Japan | 1996-2020 |
| Published registered utility model applications of Japan | 1994-2020 |

Electronic data base consulted during the international search (name of data base and, where practicable, search terms used)
JSTPlus/JMEDPlus/JST7580 (JDreamIII); CAplus/MEDLINE/EMBASE/BIOSIS/WPIDS (STN)

### C. DOCUMENTS CONSIDERED TO BE RELEVANT

| Category* | Citation of document, with indication, where appropriate, of the relevant passages | Relevant to claim No. |
|---|---|---|
| X | WO 2015/012415 A1 (UBE INDUSTRIES, LTD.) | 1-13 |
| Y | 29.01.2015 (2015-01-29) claims, paragraphs [0055], [0074], [0093], [0095], [0105], example 5 | 11, 14 |
| X | JP 2017-517505 A (CHILDREN'S MEDICAL CENTER | 14 |
| Y | CORPORATION) 29.06.2017 (2017-06-29) paragraphs [0002], [0102]-[0104] | 11, 14 |
| X | US 2014/0227766 A1 (AGENCY FOR SCIENCE, TECHNOLOGY | 14 |
| Y | AND RESEARCH) 14.08.2014 (2014-08-14) examples 22, 30 | 11, 14 |
| A | WO 2010/038873 A1 (UBE INDUSTRIES, LTD.) 08.04.2010 (2010-04-08) entire text, all drawings | 1-14 |
| A | WO 2018/021359 A1 (UBE INDUSTRIES, LTD.) 01.02.2018 (2018-02-01) entire text, all drawings | 1-14 |

☐ Further documents are listed in the continuation of Box C.   ☒ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 01 October 2020 (01.10.2020) | 13 October 2020 (13.10.2020) |

| Name and mailing address of the ISA/ | Authorized officer |
|---|---|
| Japan Patent Office 3-4-3, Kasumigaseki, Chiyoda-ku, Tokyo 100-8915, Japan | |
| | Telephone No. |

Form PCT/ISA/210 (second sheet) (January 2015)

INTERNATIONAL SEARCH REPORT
Information on patent family members

International application No.

PCT/JP2020/030614

| Patent Documents referred in the Report | Publication Date | Patent Family | Publication Date |
|---|---|---|---|
| WO 2015/012415 A1 | 29 Jan. 2015 | US 2016/0168560 A1 claims, paragraphs [0096], [0115], [0137], [0139], [0149], example 5 EP 3026108 A1 | |
| JP 2017-517505 A | 29 Jun. 2017 | US 2017/0258840 A1 paragraphs [0002], [0096]-[0098] EP 3145493 A1 WO 2015/179227 A1 | |
| US 2014/0227766 A1 | 14 Aug. 2014 | EP 2720768 A1 WO 2012/169970 A1 | |
| WO 2010/038873 A1 | 08 Apr. 2010 | US 2011/0318556 A1 entire text, all drawings EP 2354180 A1 | |
| WO 2018/021359 A1 | 01 Feb. 2018 | US 2019/0264156 A1 entire text, all drawings EP 3489342 A1 | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP SHONO62065681 A
- WO 2008084857 A
- JP HEINO7313151 A
- WO 2003054174 A
- JP 5549209 B
- JP 5460241 B
- WO 2018021368 A
- WO 2010038873 A
- JP 2011219585 A
- JP 2011219586 A
- WO 2018021367 A
- WO 2018021359 A
- WO 2009123349 A
- JP 2009057041 W

**Non-patent literature cited in the description**

- **OGATA et al.** *Journal of Fermentation and Bioengineering,* 1994, vol. 77 (1), 46-51
- **DOSH, R. H. et al.** *Science Reports,* 2019, vol. 9
- **M. MIRBAGHERI et al.** *Mater. Horiz.,* 2019, vol. 6, 45-71
- **KUROSAWA, H.** *Seibutsu Kagaku,* 2013, vol. 91 (11), 646-653